# EUROPEAN PATENT APPLICATION

(11) **EP 4 851 286 A1**
(43) Date of publication of application: **09.12.2026**
(21) Application number: 25748399.0
(22) Date of filing: 20.01.2025
(51) Int. Cl.: C12N 15/10

(54) **METHOD FOR RECOVERING NUCLEIC ACID AND KIT USED THEREFOR**

(30) Priority: 30.01.2024 JP 2024011948
(71) Applicant: Advansentinel Inc., Osaka-shi, Osaka, 541-0045 (JP)
(72) Inventor: KUROITA, Tomohiro, Osaka-shi, Osaka 541-0045 (JP); IWAMOTO, Ryo, Osaka-shi, Osaka 541-0045 (JP)
(74) Representative: Müller-Boré & Partner Patentanwälte PartG mbB
(86) International application number: PCT/JP2025/001562
(87) International publication number: WO 2025/164403

(57) **Abstract**

A method for recovering a nucleic acid present in an environmental liquid sample, comprising: a preparation step of preparing the liquid sample; a suspension step of adding a capture agent together with a cationic compound to the prepared environmental liquid sample to generate a suspension; and a recovery step of recovering the capture agent contained in the suspension, wherein the capture agent includes a glass fiber or a silica carrier, and the cationic compound has a secondary amino group or a quaternary amino group.

## Description

### [Technical Field]

The present invention relates to a method for recovering a nucleic acid and a kit used therefor.

### [Background Art]

Techniques for examining nucleic acids contained in environmental water (river water, sewage, seawater, etc.) are widely utilized in fields such as sewage surveillance and environmental DNA analysis.

For example, Japanese Unexamined Patent Application Publication No. 2023-068351 (Patent Literature 1) discloses a method for recovering a nucleic acid in a specimen, comprising a step of adsorbing the nucleic acid in the specimen onto a glass fiber suspended in water, and a step of recovering the glass fiber having the nucleic acid adsorbed thereon by filtration separation using a sheet having a fine mesh structure.

Further, the Environmental DNA Society (2020) Environmental DNA Survey and Experiment Manual Ver. 2.2 (Non-Patent Literature 1) discloses a method for filtering, separating, and extracting environmental DNA from environmental water.

### [Prior Art Literature]

### [Patent Literature]

Patent Literature 1: Japanese Unexamined Patent Application Publication No. 2023-068351

### [Non-Patent Literature]

Non-Patent Literature 1: Environmental DNA Society (2020) Environmental DNA Survey and Experiment Manual Ver. 2.2

### [Summary of the Invention]

### [Problems to be Solved by the Invention]

However, the methods disclosed in Patent Literature 1 and Non-Patent Literature 1 have a limit to the yield of nucleic acids that can be recovered, and further improvement is required.

The present invention has been made in view of the above circumstances, and an object of the present invention is to provide a method for recovering a nucleic acid with an improved yield.

### [Means for Solving the Problems]

As a result of intensive studies, the present inventors have found that the yield of a nucleic acid is improved by using a capture agent such as a glass fiber in combination with a predetermined cationic compound, and have completed the present invention.

A first aspect of the present invention is a method for recovering a nucleic acid present in an environmental liquid sample, comprising:
a preparation step of preparing the environmental liquid sample;
a suspension step of adding a capture agent together with a cationic compound to the prepared environmental liquid sample to generate a suspension; and
a recovery step of recovering the capture agent contained in the suspension,
wherein the capture agent includes a glass fiber or a silica carrier, and
the cationic compound has a secondary amino group or a quaternary amino group.

A second aspect of the present invention is a kit for recovering a nucleic acid comprising a composition used for recovering a nucleic acid contained in a liquid sample,
wherein the composition comprises:
a capture agent including a glass fiber or a silica carrier; and
a cationic compound having a secondary amino group or a quaternary amino group.

### [Effects of the Invention]

According to the present invention, it is possible to provide a method for recovering a nucleic acid with an improved yield.

### [Brief Description of the Drawings]

[FIG. 1A] FIG. 1A is a diagram showing an example of the configuration of a nucleic acid recovery kit.
[FIG. 1B] FIG. 1B is a diagram showing an example of the configuration of a nucleic acid recovery kit.
[FIG. 2A] FIG. 2A is a diagram for explaining an example of a procedure for recovering a nucleic acid using the nucleic acid recovery kit.
[FIG. 2B] FIG. 2B is a diagram for explaining an example of a procedure for recovering a nucleic acid using the nucleic acid recovery kit.
[FIG. 3] FIG. 3 is a block diagram showing a configuration of a management system according to an embodiment.
[FIG. 4] FIG. 4 is a diagram showing an example of the contents of a database.
[FIG. 5] FIG. 5 is an example of a display screen displayed on a display terminal device.
[FIG. 6] FIG. 6 is a flowchart showing an example of a process executed by the management system.
[FIG. 7] FIG. 7 is a diagram for explaining information and articles transferred in a providing system.
[FIG. 8] FIG. 8 is a sequence diagram for explaining a process executed in the providing system.
[FIG. 9] FIG. 9 is a schematic diagram for explaining assumed binding modes of a capture agent, a cationic compound, and a nucleic acid in the method for recovering a nucleic acid according to the present embodiment.
[FIG. 10] FIG. 10 is a photograph showing that a crushed membrane floats by adding BAC, a crushed membrane, PSI, and a polymer flocculant to river water.
[FIG. 11] FIG. 11 is a photograph showing that a crushed membrane floats by adding BAC, a crushed membrane, PSI, and a polymer flocculant to river water.
[FIG. 12] FIG. 12 is a photograph showing that a crushed membrane is gathered by a magnet by adding BAC, a crushed membrane, PSI, and triiron tetraoxide to river water.
[FIG. 13] FIG. 13 is a graph showing the yield of nucleic acid that can be recovered when a capture agent and BAC are added to river water. The horizontal axis represents the capture agent used and the presence or absence of BAC, and the vertical axis represents the average yield (average concentration) of the recovered nucleic acid.
[FIG. 14] FIG. 14 is a graph showing the yield of nucleic acid that can be recovered when a capture agent and BAC are added to river water. The horizontal axis represents the concentration of BAC used, and the vertical axis represents the average yield (average concentration) of the recovered nucleic acid.
[FIG. 15] FIG. 15 is a graph showing the yield of nucleic acid that can be recovered when a capture agent and a cationic compound are added to river water. The horizontal axis represents the cationic compound used and its concentration, and the vertical axis represents the average yield (average concentration) of the recovered nucleic acid.
[FIG. 16A] FIG. 16A is a graph showing the yield of nucleic acid that can be recovered when a capture agent and BAC are added to river water. The horizontal axis represents the capture agent used and the presence or absence of BAC, and the vertical axis represents the average yield (average concentration) of the recovered nucleic acid.
[FIG. 16B] FIG. 16B is a graph showing the yield of nucleic acid that can be recovered when a capture agent and BAC are added to river water. The horizontal axis represents the capture agent used and the presence or absence of BAC, and the vertical axis represents the average concentration of the recovered nucleic acid.
[FIG. 17] FIG. 17 is a graph showing the yield of nucleic acid that can be recovered when a capture agent, BAC, and a flocculant are added to river water (left graph) and the ratio of recovered DNA and RNA (right graph). The horizontal axis represents the capture agent and flocculant used, as well as the presence or absence of BAC, and the vertical axis represents the average yield of the recovered nucleic acid (left graph) or the ratio of DNA and RNA (right graph).
[FIG. 18] FIG. 18 is a graph showing the yield of nucleic acid that can be recovered when a capture agent and BAC are added to river water (left graph) and the ratio of recovered DNA and RNA (right graph). The horizontal axis represents the capture agent used and the presence or absence of BAC, and the vertical axis represents the average yield of the recovered nucleic acid (left graph) or the ratio of DNA and RNA (right graph).
[FIG. 19] FIG. 19 is a graph showing the yield of nucleic acid that can be recovered when a capture agent and BAC are added to river water. The horizontal axis represents the standing time after adding the capture agent and BAC, and the vertical axis represents the average yield (average concentration) of the recovered nucleic acid.
[FIG. 20] FIG. 20 is a graph showing the yield of nucleic acid that can be recovered when a capture agent, a cationic compound, and a flocculant are added to sewage. The horizontal axis represents the cationic compound and capture agent used and their concentrations, and the vertical axis represents the average yield (average concentration) of the recovered nucleic acid.
[FIG. 21] FIG. 21 is a graph showing the yield of nucleic acid that can be recovered when a capture agent, a cationic compound, and a flocculant are added to sewage. The horizontal axis represents the cationic compound and capture agent used and their concentrations, and the vertical axis represents the average yield (average concentration) of the recovered nucleic acid.
[FIG. 22] FIG. 22 is a graph showing the yield of nucleic acid that can be recovered when a capture agent and BAC are added to river water. The horizontal axis represents the shape of the capture agent used, and the vertical axis represents the average yield (average concentration) of the recovered nucleic acid.
[FIG. 23] FIG. 23 is a graph showing the yield of nucleic acid that can be recovered when a capture agent and a cationic compound are added to river water. The vertical axis represents the cationic compound used (final concentration 0.01%), and the horizontal axis represents the nucleic acid recovery ratio based on the control.

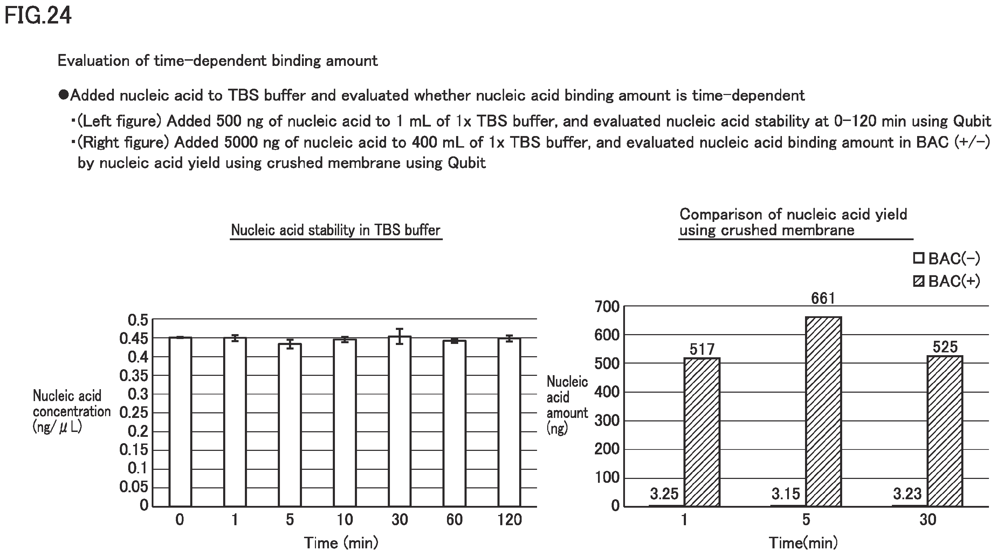

[FIG. 24] FIG. 24 is a graph showing the stability of nucleic acid in a TBS buffer (left figure) and a graph showing the yield of nucleic acid that can be recovered when a capture agent and BAC are added to a TBS buffer in which the nucleic acid is dissolved (right figure). In the left figure, the horizontal axis represents the standing time after adding the nucleic acid to the TBS buffer, and the vertical axis represents the concentration of the nucleic acid in the TBS buffer. In the right figure, the horizontal axis represents the standing time after adding the nucleic acid to the TBS buffer, and the vertical axis represents the yield of the recovered nucleic acid.
[FIG. 25] FIG. 25 is a graph showing the yield of nucleic acid that can be recovered when a capture agent and BAC are added to a clinical specimen (urine). The horizontal axis represents the capture agent used and the presence or absence of BAC, and the vertical axis represents the average concentration of the recovered nucleic acid.

### [Mode for Carrying Out the Invention]

Hereinafter, an embodiment of the present invention (hereinafter referred to as "the present embodiment") will be described. However, the present embodiment is not limited to this. In the present specification, a notation of the form "A to Z" means the upper and lower limits of the range (that is, A or more and Z or less), and when no unit is described in A and a unit is described only in Z, the unit of A and the unit of Z are the same.

### «Method for recovering nucleic acid present in environmental liquid sample>>

A first aspect of the present embodiment is a method for recovering a nucleic acid present in an environmental liquid sample, comprising:
a preparation step of preparing the environmental liquid sample;
a suspension step of adding a capture agent together with a cationic compound to the prepared environmental liquid sample to generate a suspension; and
a recovery step of recovering the capture agent contained in the suspension,
wherein the capture agent includes a glass fiber or a silica carrier, and the cationic compound has a secondary amino group or a quaternary amino group.

### <Preparation step>

In this step, an environmental liquid sample is prepared. In the present embodiment, the "environmental liquid sample" means a liquid sample derived from a substance present in a natural environment or an artificial environment. Examples of those present in a natural environment include river water, seawater, lake water, soil, compost, sludge, and the like. Examples of those present in an artificial environment include sewage, industrial wastewater, wastewater from livestock facilities, oral drain wastewater, tap water, and the like. When what exists in the natural environment or the artificial environment is a liquid, the liquid can directly be the environmental liquid sample. When what exists in the natural environment or the artificial environment is a solid, a suspension obtained by suspending it in distilled water or the like can be the environmental liquid sample. As the environmental liquid sample, a sample collected from a natural environment or an artificial environment may be used as it is, or a sample subjected to pretreatment such as removal of insoluble components may be used.

In one aspect of the present embodiment, the environmental liquid sample may contain at least one selected from the group consisting of river water, seawater, lake water, and sewage. Further, the environmental liquid sample may consist of at least one selected from the group consisting of river water, seawater, lake water, and sewage. The volume of the environmental liquid sample is not particularly limited, but may be, for example, 10 mL or more and 2000 mL or less, or 400 mL or more and 1000 mL or less.

In another aspect of the present embodiment, in the preparation step, the environmental liquid sample does not have to contain a cationic compound. This is because, in the present embodiment, the cationic compound is used to promote binding between the capture agent and the nucleic acid.

The environmental liquid sample may contain a nucleic acid to be examined. The nucleic acid may be DNA, RNA, or both. The nucleic acid to be examined is not particularly limited, and may be of plant origin, animal origin (for example, fish, etc.), or microorganism origin.

### <Suspension step>

In this step, a capture agent is added together with a cationic compound to the prepared environmental liquid sample to generate a suspension. In the present embodiment, "adding a capture agent together with a cationic compound" means that the capture agent and the cationic compound are used together in this step, rather than in other steps. The order of adding the capture agent and the cationic compound is not particularly limited. The capture agent may be added to the environmental liquid sample first, and then the cationic compound may be added. The cationic compound may be added to the environmental liquid sample first, and then the capture agent may be added. Alternatively, the capture agent and the cationic compound may be simultaneously added to the environmental liquid sample. In one aspect of the present embodiment, a composition (mixture) containing the capture agent and the cationic compound may be added to the environmental liquid sample.

This step may further include mixing (for example, inverting to mix) the generated suspension.

In one aspect of the present embodiment, it is preferable to perform the suspension step and a recovery step described later before the degradation of the nucleic acid in the environmental liquid sample proceeds, particularly immediately after the environmental liquid sample is collected and prepared. From this viewpoint, in the suspension step, the time from the start to the end (from moving to the suspension step until starting the recovery step) may be 1 minute or more and 10 minutes or less, or 1 minute or more and 60 minutes or less.

In the present embodiment, the "capture agent" means a reagent that adsorbs and recovers a nucleic acid contained in an environmental liquid sample. The capture agent includes a glass fiber or a silica carrier. The capture agent may include a glass fiber, or may consist of a glass fiber.

Examples of the material of the glass fiber include borosilicate glass, soda-lime glass, quartz glass, and the like. In one aspect of the present embodiment, the material of the glass fiber may include borosilicate glass, or may consist of borosilicate glass.

The shape of the glass fiber may be a powder form, a sheet form like filter paper, or a piece form. In one aspect of the present embodiment, the capture agent may include a powdery glass fiber, or may consist of a powdery glass fiber. The arithmetic average fiber length of the powdery glass fiber may be 20 µm or more and 500 µm or less, or 100 µm or more and 300 µm or less. The arithmetic average fiber length can be obtained by measuring with, for example, Valmet FS5 (manufactured by Valmet).

The glass fiber may be manufactured by a known method, or a commercially available product may be purchased. Examples of commercially available products of the glass fiber include products described in the examples below. The powdery glass fiber can be obtained, for example, by crushing a commercially available glass filter paper using stainless steel beads (see the examples below).

The shape of the silica carrier may be a powder form, a sheet form, a gel form, or a fibrous form. The average particle size of the powder-form silica carrier may be 5 µm or more and 200 µm or less. The average particle size of the gel-form silica carrier may be 5 µm or more and 100 µm or less. The arithmetic average fiber length in the fibrous silica carrier may be 20 µm or more and 500 µm or less, or 100 µm or more and 300 µm or less. The arithmetic average fiber length can be measured by, for example, Valmet FS5 (manufactured by Valmet).

The silica carrier may be manufactured by a known method, or a commercially available product may be purchased. Examples of commercially available products of the silica carrier include products described in the examples below. The fibrous silica carrier can be obtained, for example, by crushing a commercially available silica filter paper using stainless steel beads (see the examples below).

The concentration of the capture agent may be 0.0005 w/v% or more and 1 w/v% or less, or 0.0025 w/v% or more and 0.1 w/v% or less, based on the suspension.

In the present embodiment, the "cationic compound" means a compound that acts as a cation when dissolved in water. The cationic compound has a secondary amino group or a quaternary amino group. In the present embodiment, in the quaternary amino group, two substituents bonded to a nitrogen atom may be bonded to each other to form a ring structure (for example, an imidazole ring, a pyridine ring). That is, in the present embodiment, the "quaternary amino group" may include an imidazole group and a pyridinium group. In one aspect of the present embodiment, the cationic compound may have an imino group.

In one aspect of the present embodiment, the cationic compound may further contain a hydrophobic group (for example, a straight-chain alkyl group having 10 to 18 carbon atoms, etc.) or an aromatic ring. In another aspect of the present embodiment, the cationic compound may further have a straight-chain alkyl group having 10 to 18 carbon atoms.

In another aspect of the present embodiment, the cationic compound may include a compound having a quaternary amino group and having a straight-chain alkyl group having 10 or more carbon atoms (preferably a straight-chain alkyl group having 10 or more and 18 or less carbon atoms), or a compound having a secondary amino group and having a bactericidal activity. Here, "bactericidal activity" means an action to kill microorganisms.

Examples of the cationic compound having a secondary amino group include chlorhexidine gluconate, alkyldiaminoethylglycine hydrochloride, polyhexamethylene biguanide, N-methyldodecylamine, and the like. Examples of the cationic compound having a quaternary amino group include benzalkonium chloride, domiphen bromide, hexadecyltrimethylammonium bromide, 1-decyl-3-methylimidazolium chloride, 1,3-didecyl-2-methylimidazolium, tetradecyltrimethylammonium, cetylpyridinium, dodecyltrimethylammonium, trimethyloctadecylammonium, benzyldimethyldodecylammonium, 1-dodecylpyridinium chloride, benzyldimethylhexadecylammonium chloride, cetylpyridinium bromide, and didecyldimethylammonium. Examples of the cationic compound having an imino group include chlorhexidine gluconate.

In one aspect of the present embodiment, the cationic compound may include at least one selected from the group consisting of benzalkonium chloride, chlorhexidine gluconate, alkyldiaminoethylglycine hydrochloride, polyhexamethylene biguanide, N-methyldodecylamine, domiphen bromide, hexadecyltrimethylammonium bromide, 1-decyl-3-methylimidazolium chloride, 1,3-didecyl-2-methylimidazolium, tetradecyltrimethylammonium, cetylpyridinium, dodecyltrimethylammonium, trimethyloctadecylammonium, benzyldimethyldodecylammonium, 1-dodecylpyridinium chloride, benzyldimethylhexadecylammonium chloride, cetylpyridinium bromide, and didecyldimethylammonium. The cationic compound may include benzalkonium chloride, or may consist of benzalkonium chloride.

The concentration of the cationic compound may be 0.0001 w/v% or more and 0.05 w/v% or less, 0.0005 w/v% or more and 0.05 w/v% or less, or 0.001 w/v% or more and 0.01 w/v% or less, based on the suspension. When the cationic compound contains benzalkonium chloride, the concentration of the benzalkonium chloride is preferably 0.0005 w/v% or more and 0.05 w/v% or less, and more preferably 0.001 w/v% or more and 0.01 w/v% or less, based on the suspension.

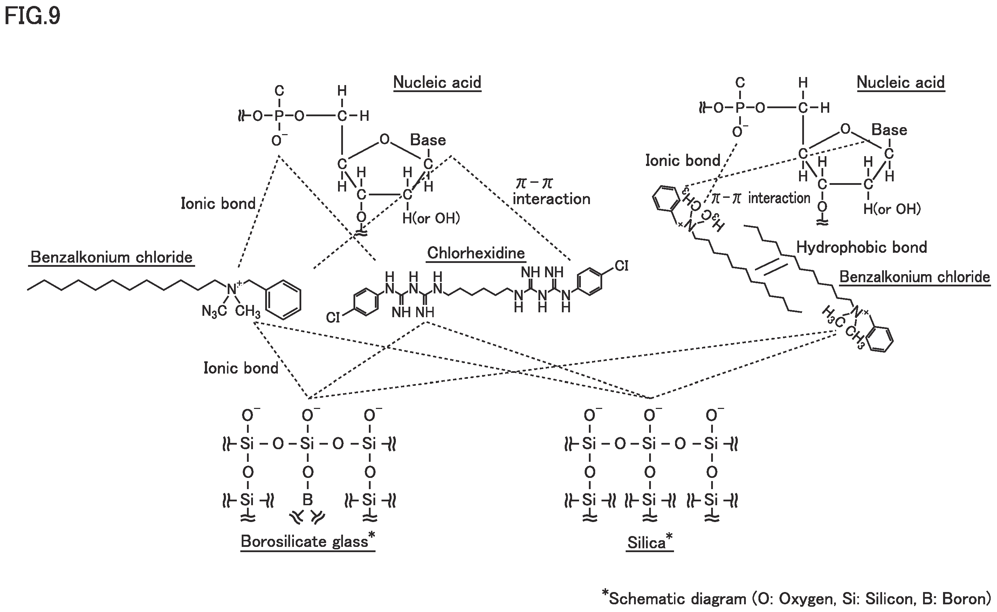

Conventionally, it has been known to add benzalkonium chloride as a disinfectant to an environmental liquid sample. However, it was not known that the yield of a nucleic acid was significantly improved when a cationic compound such as benzalkonium chloride was added to an environmental liquid sample together with a capture agent, which the present inventors found for the first time. The specific mechanism by which the yield of a nucleic acid is improved is not clear, but first, a positively charged cationic compound forms an ionic bond with a negatively charged base of a nucleic acid or surface of a capture agent, and then a plurality of cationic compounds forming ionic bonds form hydrophobic bonds or π-π interactions with each other (by forming a bridge structure), whereby the present inventors believe that the adsorption amount of the nucleic acid to the capture agent is improved (FIG. 9). In view of such an action of the cationic compound, the cationic compound can also be understood as a "promoter that promotes the binding between the capture agent and the nucleic acid."

Further, in the method for recovering a nucleic acid according to the present embodiment, it is considered that the recovery rate of a nucleic acid is improved. Therefore, it becomes possible to recover trace amounts of nucleic acid species contained in an environmental sample, which could not be recovered by the conventional technology. Since it becomes possible to recover trace amounts of nucleic acid species contained in environmental samples, it is expected to increase the biological species that can be identified in metabarcoding and to identify microorganisms present in small amounts.

In one aspect of the present embodiment, the mass ratio of the capture agent to the cationic compound (mass of capture agent/mass of cationic compound) in the suspension may be 0.25 or more and 250 or less, or 1.25 or more and 62.5 or less. In another aspect of the present embodiment, the cationic compound is benzalkonium chloride, and the mass ratio of the capture agent to the benzalkonium chloride in the suspension is preferably 0.25 or more and 250 or less, and more preferably 1.25 or more and 62.5 or less.

In one aspect of the present embodiment, the pH of the suspension is not particularly limited as long as the effects of the present invention are exhibited. The pH of the suspension may be 5.3 or more and 9.3 or less, or 6 or more and 8 or less. In order to set the pH of the suspension within the above range, a pH adjusting agent may be added in the suspension step.

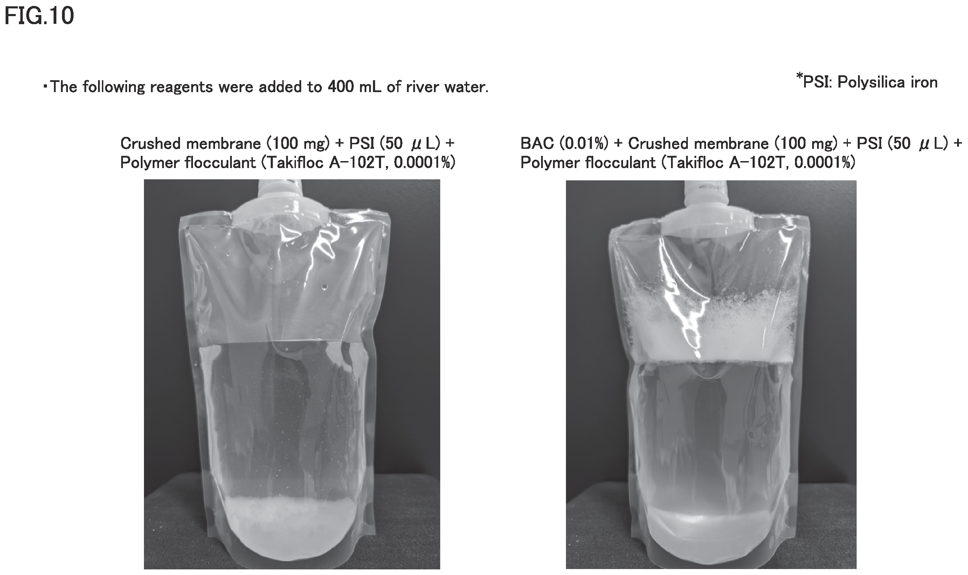

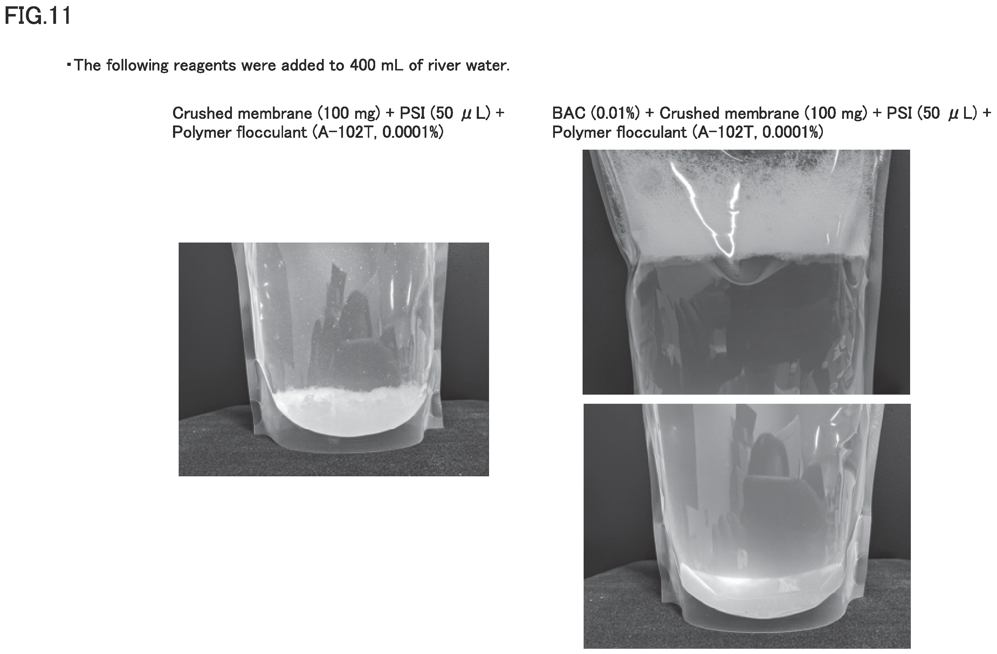

In one aspect of the present embodiment, the method may further include adding a flocculant in the suspension step. Here, the "flocculant" means a reagent that agglomerates the capture agent by being added to the suspension. By adding the flocculant, the capture agent floats in an agglomerated state in the upper part of the suspension (for example, FIGS. 10 and 11). Therefore, when the capture agent is recovered by filtration, there is a tendency to suppress clogging of the filter and suppress a decrease in filtration speed.

Examples of the flocculant include polyaluminum chloride (PAC), polysilica iron compounds (PSI), and polymer flocculants. In the present embodiment, the flocculant may contain at least one selected from the group consisting of polyaluminum chloride, polysilica iron compounds, and polymer flocculants. Examples of the polymer flocculants include N-131 (nonionic, molecular weight 1600), A-102T (weakly anionic, molecular weight 1700), A-108T (moderately anionic, molecular weight 1900), and A-133 (strongly anionic, molecular weight 700) manufactured by Taki Chemical Co., Ltd. The flocculant may be manufactured by a known method, or a commercially available product may be purchased.

The flocculant may be in a powder form or a liquid form.

The concentration of the PAC or the PSI may be 0.001 v/v% or more and 0.2 v/v% or less, or 0.002 v/v% or more and 0.0125 v/v% or less, based on the suspension. The concentration of the polymer flocculant may be 0.00001 w/v% or more and 0.001 w/v% or less, or 0.00005 w/v% or more and 0.0005 w/v% or less, based on the suspension.

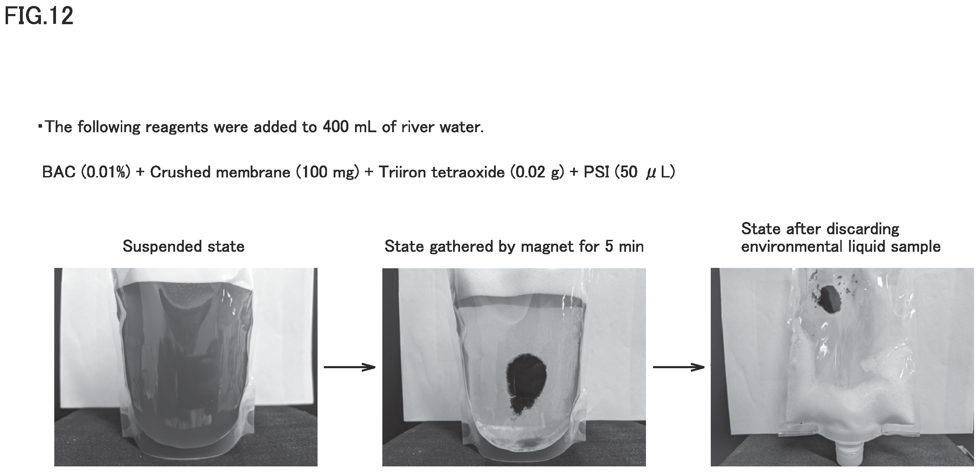

In one aspect of the present embodiment, when a flocculant is added, the method may further include adding triiron tetraoxide in the suspension step. By adding the triiron tetraoxide, the capture agent agglomerates together with the triiron tetraoxide. Therefore, by applying magnetic force, the capture agent can be collected at a predetermined position (for example, FIG. 12).

The triiron tetraoxide may be in a powder form or a liquid form.

The concentration of the triiron tetraoxide may be 0.0005 w/v% or more and 0.05 w/v% or less, or 0.0025 w/v% or more and 0.025 w/v% or less, based on the suspension.

### <Recovery step>

In this step, the capture agent contained in the suspension is recovered. The method for recovering the capture agent from the suspension is not particularly limited, and a known method may be performed. In one aspect of the present embodiment, in the recovery step, the capture agent may be recovered by filtering the suspension. The method of filtration may be gravity filtration, vacuum filtration, or pressure filtration. The filter paper used when filtering the suspension may be any filter paper as long as the capture agent does not pass through the filter paper. In one aspect of the present embodiment, the mesh size of the filter paper may be, for example, 1 µm or more and 200 µm or less, 1 µm or more and less than 180 µm, or 1 µm or more and 175 µm or less. In another aspect of the present embodiment, the mesh size of the filter paper may be, for example, 20 µm or more and 200 µm or less. In another aspect of the present embodiment, when a flocculant and triiron tetraoxide are added in the suspension step, the capture agent may be recovered by applying a magnetic force. At this time, the capture agent forms a floc together with the flocculant and the triiron tetraoxide.

### <Other steps>

In one aspect of the present embodiment, the method for recovering a nucleic acid may further include an extraction step of extracting the nucleic acid from the capture agent recovered in the recovery step. The method of extracting the nucleic acid from the capture agent is not particularly limited, and it can be recovered by a known method. The extraction step may be performed using, for example, a commercially available kit for nucleic acid extraction used in the examples described later.

### «Kit used for method for recovering nucleic acid>>

A kit used for carrying out the method for recovering a nucleic acid described above will be described.

### <Configuration of Kit 1>

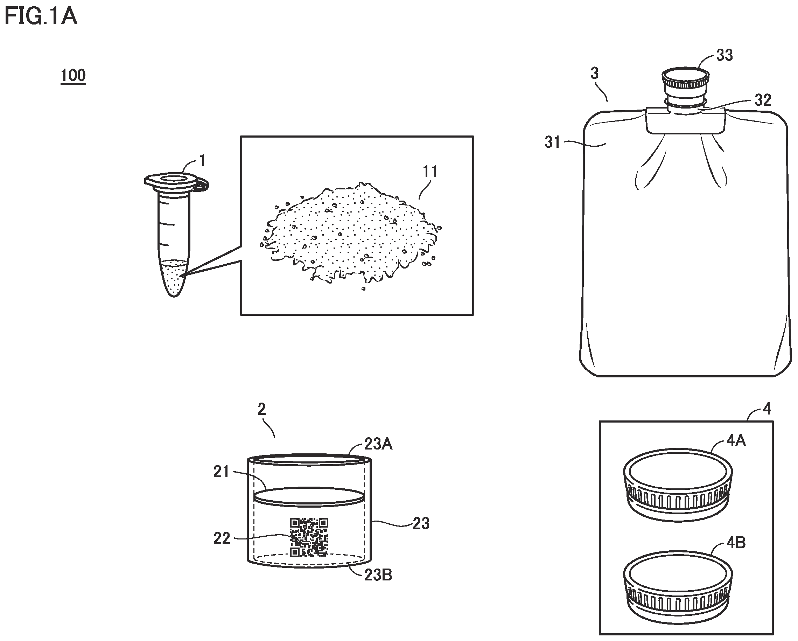

FIG. 1A is a diagram showing an example of the configuration of a nucleic acid recovery kit. The nucleic acid recovery kit 100 includes a composition 11 contained in a container 1, a filter unit 2, a sampling bag 3, and caps 4. By using the nucleic acid recovery kit 100, the user can recover nucleic acids from a liquid sample (for example, an environmental liquid sample) by the method described above.

The composition 11 is contained in the container 1. The composition 11 contains a capture agent and a cationic compound. The capture agent is the same as the capture agent used in the method for recovering a nucleic acid described above, and means a reagent that adsorbs and recovers nucleic acids contained in a liquid sample. The capture agent includes a glass fiber or a silica carrier. The capture agent may include a glass fiber, or may consist of a glass fiber. The capture agent may be in a powder form, a sheet form, or a gel form. The cationic compound is the same as the cationic compound used in the method for recovering a nucleic acid described above, and means a compound that acts as a cation when dissolved in water. The cationic compound has a secondary amino group or a quaternary amino group.

The filter unit 2 includes a filter 21 and a housing 23. In the filter unit 2, the filter 21 is provided inside the cylindrical housing 23. The filter 21 has pores sized to allow the liquid to pass therethrough while not allowing the capture agent to pass therethrough. One end of the housing 23 has an opening 23A for introducing a liquid, and the liquid introduced from the opening 23A passes through the filter 21 and is discharged from an opening 23B provided at the other end of the housing 23. An identifier 22 is attached to the outside of the side wall of the housing 23. The identifier 22 is, for example, a QR code (registered trademark) or a barcode. In the identifier 22, identification information used to uniquely distinguish a predetermined filter unit from other filter units is recorded. The identification information is, for example, a code in which letters, numbers, and/or symbols are combined.

The sampling bag 3 includes a main body portion 31, an opening portion 32, and a cap 33. The user can put the liquid sample and the composition 11 in the sampling bag 3 and mix them. When the opening portion 32 is closed by the cap 33, the inside of the sampling bag 3 is in an airtight state. The opening portion 32 can be connected to the opening 23A of the filter unit 2.

The caps 4 include a cap 4A and a cap 4B. The cap 4A has a shape capable of connecting to the opening 23A of the filter unit 2, and the cap 4B is capable of connecting to the opening 23B of the filter unit 2. When the opening 23A and the opening 23B are closed by the cap 4A and the cap 4B, the inside of the filter unit 2 is in an airtight state. Further, when the cap 4A is coupled to the opening 23A, a space having a predetermined size is formed inside the filter unit 2 by the cap 4A, the filter 21, and the housing 23.

In the nucleic acid recovery kit 100, the composition 11 may be provided in a state accommodated in the container 1, or may be provided in a state accommodated inside the sampling bag 3.

As another aspect of the nucleic acid recovery kit, the nucleic acid recovery kit includes the composition 11 accommodated in the container 1. According to the kit, by mixing a liquid sample put in a general container (for example, a beaker) and the composition 11, nucleic acids in the liquid sample bind to the capture agent via the cationic compound. The user can recover the capture agent bound to the nucleic acids from the suspension of the liquid sample and the composition by a general filtration device.

As another aspect of the kit for carrying out the method for recovering a nucleic acid described above, the nucleic acid recovery kit includes the filter unit 2, the sampling bag 3, and the caps 4. For example, it is assumed that an amount of the composition 11 equal to or more than the amount required for one extraction step is provided to the user. In such a case, the user prepares the filter unit 2, the sampling bag 3, and the caps 4 according to the number of samples. Therefore, as a kit used for the above-described method for recovering a nucleic acid, a nucleic acid recovery kit including the filter unit 2, the sampling bag 3, and the caps 4 may be provided to the user separately from the composition 11.

### <Configuration of Kit 2>

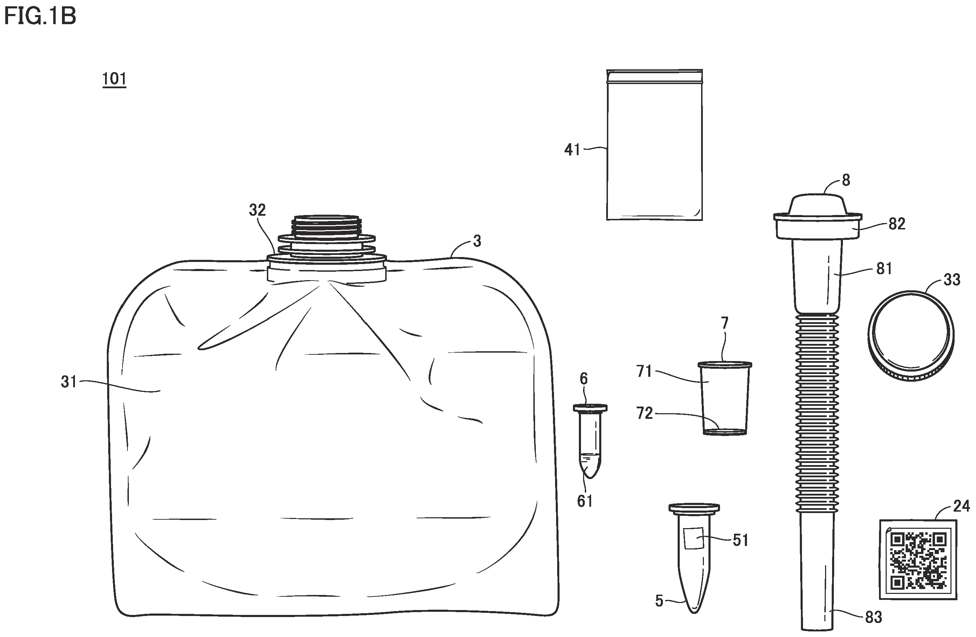

FIG. 1B is a diagram showing an example of a nucleic acid recovery kit having a configuration different from the nucleic acid recovery kit shown in FIG. 1A. The nucleic acid recovery kit 101 includes a sampling bag 3, a capture agent 51 contained in a container 5, an additive liquid 61 contained in a container 6, a filter unit 7, a discharge unit 8, a seal 24, and a storage bag 41. By using the nucleic acid recovery kit 101, the user can recover nucleic acids from a liquid sample (for example, an environmental liquid sample) by the method described above. In addition, the same or corresponding parts as those of the nucleic acid recovery kit 100 are denoted by the same reference numerals, and overlapping descriptions will not be repeated.

The capture agent 51 contained in the container 5 is the same as the capture agent used in the method for recovering a nucleic acid described above, and means a reagent that adsorbs and recovers nucleic acids contained in a liquid sample. The capture agent 51 includes a glass fiber or a silica carrier. The capture agent 51 may include a glass fiber, or may consist of a glass fiber. The capture agent 51 may be in a powder form, a sheet form, or a gel form.

The additive liquid 61 contained in the container 6 contains a cationic compound. The cationic compound is the same as the cationic compound used in the method for recovering a nucleic acid described above, and means a compound that acts as a cation when dissolved in water. The cationic compound has a secondary amino group or a quaternary amino group.

In addition, the additive liquid 61 may further contain a flocculant. Here, the "flocculant" means a reagent that agglomerates the capture agent 51 by being added to the suspension. By adding the flocculant, the capture agent 51 floats in an agglomerated state in the upper part of the suspension (for example, FIGS. 10 and 11). Therefore, when the capture agent 51 is recovered by filtration, there is a tendency to suppress clogging of the filter 72 and suppress a decrease in filtration speed.

The filter unit 7 includes a housing 71 and a filter 72. In the filter unit 7, the filter 72 is provided inside the cylindrical housing 71. The filter 72 has pores sized to allow the liquid to pass therethrough while not allowing the capture agent 51 to pass therethrough. The liquid introduced from one end of the cylindrical housing 71 passes through the filter 72 and is discharged from an opening provided at the other end of the housing 71. The capture agent 51 recovered on the filter 72 is accommodated in the storage bag 41 together with the filter unit 7 and stored in a refrigerator.

The discharge unit 8 is used to discharge the liquid in the sampling bag 3. The discharge unit 8 includes an accommodating portion 81, a holed cap 82, and a nozzle portion 83. The accommodating portion 81 is configured to be able to accommodate the filter unit 7. The holed cap 82 has a shape capable of connecting to the opening portion 32 of the sampling bag 3. In a state where the sampling bag 3 and the discharge unit 8 are coupled, the liquid in the sampling bag 3 is discharged from the opening portion 32, then passes through the accommodating portion 81, and is discharged from the nozzle portion 83.

An identifier is printed on the seal 24. The identifier is, for example, a QR code (registered trademark) or a barcode. The seal 24 is attached to the storage bag 41 in a state where the identifier can be read. In the identifier, identification information used to uniquely distinguish a filter unit in a predetermined storage bag from a filter unit in another storage bag is recorded. The identification information is, for example, a code in which letters, numbers, and/or symbols are combined.

In the nucleic acid recovery kit 101, the capture agent 51 may be provided in a state accommodated in the sampling bag 3.

As another aspect of the kit for carrying out the method for recovering a nucleic acid described above, the nucleic acid recovery kit 101 includes the filter unit 7, the sampling bag 3, and the storage bag 41. For example, it is assumed that an amount of the capture agent 51 and the additive liquid 61 equal to or more than the amount required for one extraction step is provided to the user. In such a case, the user prepares the filter unit 7, the sampling bag 3, and the storage bag 41 according to the number of samples. Therefore, as a kit used for the above-described method for recovering a nucleic acid, a nucleic acid recovery kit including the filter unit 7, the sampling bag 3, and the storage bag 41 may be provided to the user separately from the capture agent 51 and the additive liquid 61.

### <<How to use the nucleic acid recovery kit>>

Subsequently, a method of recovering nucleic acids from a liquid sample using the nucleic acid recovery kit will be described.

### <How to use 1>

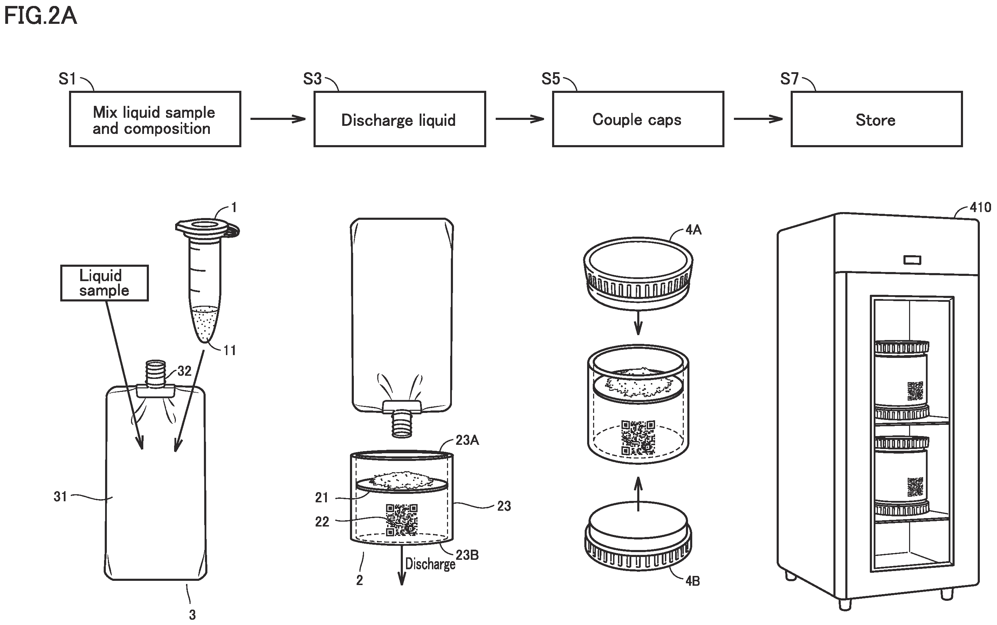

FIG. 2A is a diagram for explaining a procedure for recovering nucleic acids using the nucleic acid recovery kit 100.

In step S1, the user puts the collected liquid sample and the composition 11 into the sampling bag 3 to form a suspension. The capture agent is insoluble in water, and the cationic compound is soluble in water. After that, the user closes the opening portion 32 with the cap 33. After closing the opening portion 32, the user mixes the suspension. By this operation, the nucleic acids in the liquid sample bind to the capture agent via the cationic compound.

In step S3, the user removes the cap 33 from the sampling bag 3, and connects the opening 23A of the filter unit 2 and the opening portion 32 of the sampling bag 3. After connecting the filter unit 2 to the sampling bag 3, the user discharges the contents of the sampling bag 3 to the outside of the sampling bag 3 through the filter unit 2. Since the filter 21 allows the liquid to pass therethrough but not the capture agent, the capture agent bound to the nucleic acids remains on the filter 21.

In step S5, in a state where the capture agent remains on the filter 21, the user removes the filter unit 2 and the sampling bag 3, and closes the opening 23A and the opening 23B of the filter unit 2 with the cap 4A and the cap 4B, respectively. By this operation, the capture agent bound to the nucleic acids is accommodated in a predetermined space inside the filter unit 2.

In step S7, the user stores the filter unit 2 accommodating the capture agent in a storage 410. The filter unit 2 and the caps 4 serve as a container for accommodating the capture agent bound to the nucleic acids.

### <How to use 2>

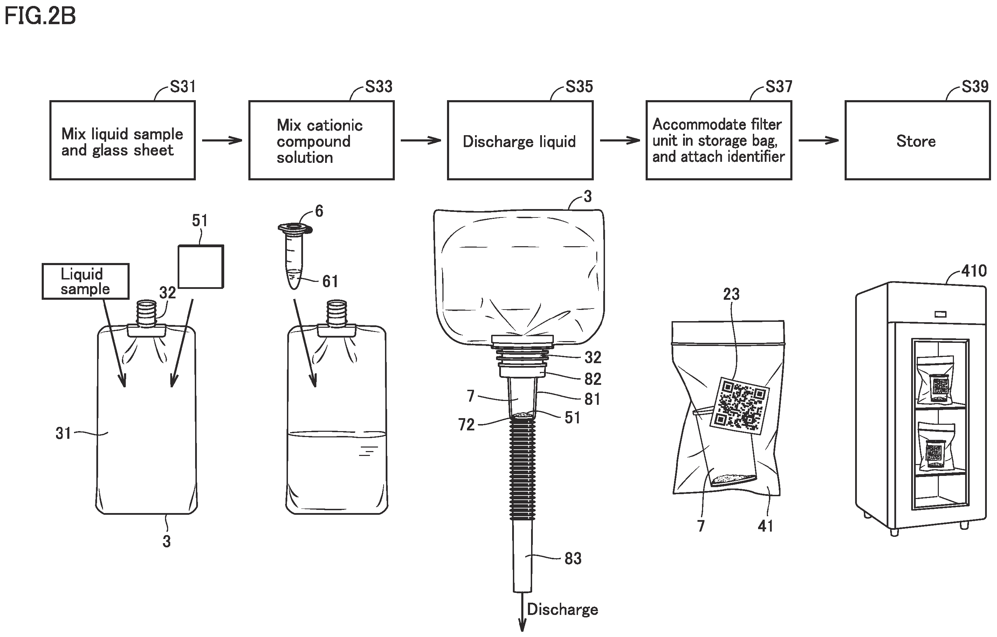

Subsequently, a method of recovering nucleic acids from a liquid sample using the nucleic acid recovery kit 101 will be described. FIG. 2B is a diagram for explaining a procedure for recovering nucleic acids using the nucleic acid recovery kit 101.

In step S31, the user puts the collected liquid sample and the capture agent 51 into the sampling bag 3 to form a mixed liquid. The capture agent is insoluble in water. After that, the user closes the opening portion 32 with the cap 33. After closing the opening portion 32, the user stirs the mixed liquid. By this operation, the capture agent 51 is crushed into fine pieces and diffused in the mixed liquid.

In step S33, the user removes the cap 33 from the sampling bag 3, and adds the additive liquid 61 to the sampling bag 3 to form a suspension. After that, the user closes the opening portion 32 with the cap 33. After closing the opening portion 32, the user mixes the suspension. By this operation, the nucleic acids in the liquid sample bind to the capture agent 51 via the cationic compound contained in the additive liquid 61.

In step S35, the user removes the cap 33 from the sampling bag 3, and in a state where the filter unit 7 is accommodated in the accommodating portion 81, connects the holed cap 82 and the opening portion 32 of the sampling bag 3. The user discharges the contents of the sampling bag 3 to the outside of the sampling bag 3 through the filter unit 7. Since the filter 72 allows the liquid to pass therethrough but not the capture agent 51, the capture agent 51 bound to the nucleic acids remains on the filter 72.

In step S37, in a state where the capture agent 51 is recovered by the filter 72, the user takes out the filter unit 7 from the accommodating portion 81 and accommodates the filter unit 7 in the storage bag 41. Thereby, it is possible to prevent nucleic acids derived from anything other than the used liquid sample from adhering to the capture agent 51. After accommodating the filter unit 7 in the storage bag 41, the seal 24 is attached to the storage bag 41.

In step S39, the user stores the storage bag 41 accommodating the filter unit 7 containing the capture agent 51 in the storage 410.

When the nucleic acids recovered by the nucleic acid recovery kit 100 and the nucleic acid recovery kit 101 described above are subjected to analysis, the user adds the capture agent taken out from the filter unit 2 and the capture agent 51 retained on the filter 72 of the filter unit 7 to an elution liquid. The elution liquid is, for example, water and a buffer solution. Since the nucleic acids bound to the capture agent via the cationic compound dissolve in the elution liquid, the user can subject the elution liquid in which the nucleic acids are dissolved to a desired analysis.

By using the nucleic acid recovery kit 100 and the nucleic acid recovery kit 101 described above, the user can recover nucleic acids without using an apparatus such as a centrifuge. Therefore, the user can recover nucleic acids with the kit at the place where the liquid sample is collected. Thereby, the labor and cost involved in transporting the liquid sample can be reduced.

Further, the recovered nucleic acids are stored in a state bound to the capture agent. Since the liquid sample can be stored by removing substances other than nucleic acids, storage of the capture agent can be realized in a smaller space than storing the liquid sample.

Furthermore, in the recovery method using the above-described kit, the user can accommodate the capture agent in the filter unit 2 and the filter unit 7 without directly touching the capture agent diffused in the liquid sample. Therefore, it is possible to prevent nucleic acids other than the nucleic acids contained in the liquid sample from binding to the capture agent.

In addition, an identifier indicating identification information is attached to the storage bag 41 accommodating the filter unit 2 and the filter unit 7 of the kit. That is, the capture agent to which the nucleic acids are bound is accommodated in a container to which an identifier is attached. By registering the identification information and the information of the liquid sample in association with each other in a server, the user can efficiently manage the recovered nucleic acids and their information. The management method of the capture agent accommodated in the filter unit 2 and the capture agent 51 accommodated in the storage bag 41 together with the filter unit 7 will be described later.

In step S3 and step S35 described above, the discharging of the contents of the sampling bag 3 may be by gravity drop, or the user may crush the sampling bag 3 to apply pressure inside the sampling bag 3 to discharge. Also, the contents of the sampling bag 3 may be sucked from the outside of the sampling bag 3. For example, in this case, the nozzle portion 83 of the discharge unit 8 may have a connection port having a shape connectable to a suction filter flask capable of pressure reduction, or the discharge unit 8 may not include the nozzle portion 83, and the accommodating portion 81 may have a connection port having a shape connectable to a suction filter flask capable of pressure reduction. Also, the opening 23B of the filter unit 2 has a shape capable of being connected to a suction filter flask capable of pressure reduction. By discharging the contents in the sampling bag 3 by suction, the time required for recovering the nucleic acids can be shortened.

### ≪Sample Management System≫

In research targeting environmental samples, researchers may need to travel to the site to collect samples. Therefore, it takes time and cost for researchers to travel to the site.

In addition, when a researcher uses the analysis result of a sample for research, information on the sample may be required. The information on the sample includes, for example, at least one of a location where the sample was collected, a date and time when the sample was collected, and a person who collected the sample. However, because a sample and its sample information are not stored in association with each other, a researcher may not be able to use the analysis result of the sample for research.

Furthermore, there are cases where environmental conservation activities are carried out through a feedback of analysis results to ordinary citizens by not only researchers but also ordinary citizens collecting environmental samples and sending the samples to a research institute. In such a case, a technology is required that enables even citizens unfamiliar with handling samples to easily acquire information on a sample and store it together with the sample.

Therefore, in the container management system according to the present embodiment, a sample and its sample information are managed in association with identification information of an identifier attached to a container in which the sample is accommodated. According to the management system, a researcher can easily acquire information on a target sample.

In addition, the container management system according to the present embodiment can centrally store collected samples and their sample information together. Therefore, by using the management system, a researcher can utilize samples and their sample information stored in the management system by others. As a result, a researcher can acquire a sample collected at a desired location and its information without actually going to the site, and use the sample for research.

In the description of the present embodiment, the sample is a capture agent to which nucleic acids are bound via a cationic compound. In this case, the sample information refers to information on a liquid sample that contained the nucleic acids bound to the capture agent. When nucleic acids are recovered using the nucleic acid recovery kit 100 described above, the filter unit 2 and the caps 4 serve as a container for accommodating the sample. Also, when nucleic acids are recovered using the nucleic acid recovery kit 101 described above, the storage bag 41 accommodating the capture agent 51 recovered on the filter 72 of the filter unit 7 corresponds to the container accommodating the sample in the present embodiment.

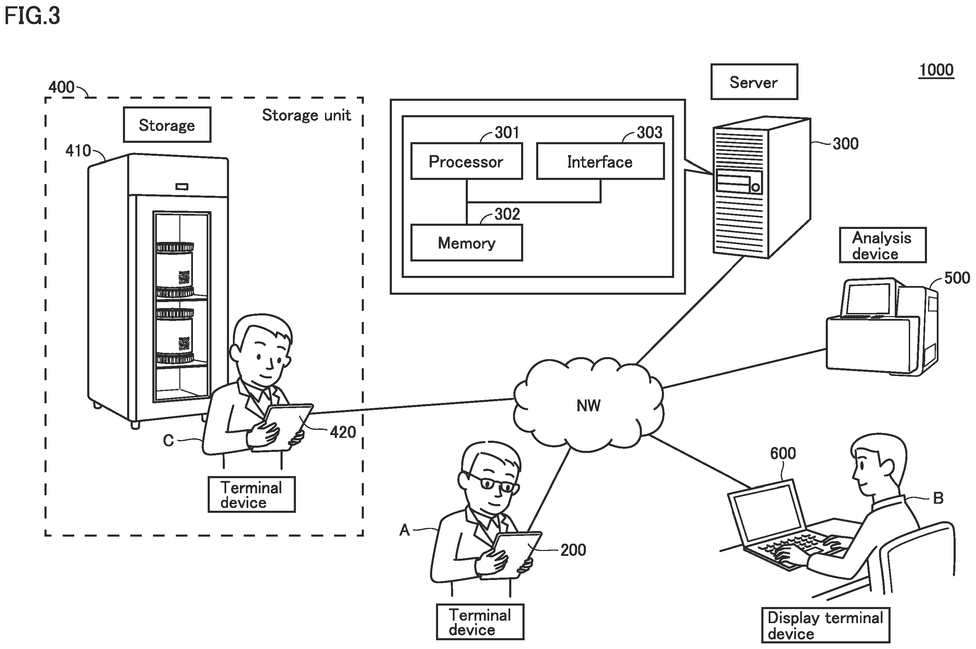

A system for managing nucleic acid samples for which a recovery method is guaranteed will be described. FIG. 3 is a block diagram showing a configuration of a management system 1000 according to an embodiment. In a general aspect, the management system 1000 is configured to allow a user B to use a sample collected by a sample collector A, sample information, and a sample analysis result. As shown in FIG. 3, the management system 1000 includes a terminal device 200, a server 300, a storage unit 400, an analysis device 500, and a display terminal device 600.

The terminal device 200, the server 300, the terminal device 420 of the storage unit 400, the analysis device 500, and the display terminal device 600 are connected to each other via a network NW. The network NW includes, for example, the Internet, a WAN (Wide Area Network), or a LAN (Local Area Network).

The terminal device 200 transmits information on a sample collected by the sample collector A to the server 300 via the network NW. At that time, the sample collector A reads identification information of a container accommodating the sample, and transmits the identification information and information on the sample accommodated in the container to the server. The terminal device 200 includes, for example, a scanner or a camera for acquiring identification information, a keyboard or a touch panel for a user to input sample information, and an antenna for acquiring position information indicating where the sample was collected.

The server 300 receives information from devices connected via the network NW, registers it in a database, and transmits the information to the devices upon request. The server 300 includes, as main components, a processor 301, a memory 302, and an interface 303. These components are connected to each other by a data bus.

The processor 301 is an example of an electric circuit, and controls the operation of the server 300 by executing a given program. The program executed by the processor 301 may be stored in the memory 302, or may be stored in a storage device (not shown) outside the server 300. The processor 301 is, for example, a CPU (Central Processing Unit).

The memory 302 can store a database and programs executed by the processor 301. The database records identification information of a container accommodating a sample, sample information, and an analysis result of the sample in association with each other. The memory 302 includes volatile memory (e.g., RAM (Random Access Memory)) and non-volatile memory (e.g., ROM (Read Only Memory), hard disk drive, and solid state drive). The above program may be stored in an external storage device accessible by the processor 301.

An interface (I/F) 303 is an interface for exchanging various data between the processor 301 and devices connected to the I/F 303. The I/F 303 is realized by, for example, a terminal block, a connector, and a network adapter. Data transfer via the I/F 303 may be performed via wireless such as Bluetooth (registered trademark) or wireless LAN, or via wired using USB (Universal Serial Bus) or the like. The server 300 may receive analysis results acquired by a device other than the analysis device 500 via the I/F 303.

The storage unit 400 stores containers accommodating samples collected by the sample collector A in a predetermined area. The storage unit 400 includes a storage 410 and a terminal device 420. The storage 410 has an accommodating section including one or more accommodating areas. Each accommodating area is assigned an area number. In the storage unit, an operator C accommodates a container received from the sample collector A in a predetermined accommodating area. The terminal device 420 includes a scanner or a camera for acquiring identification information. The operator C acquires identification information of the container using the terminal device 420 when accommodating the received container in a predetermined accommodating area. The operator C transmits the identification information of the container and an area number of the accommodating area in which the container is accommodated to the server 300 through the terminal device 420. The server 300 that has received the identification information of the sample container and the area number of the accommodating area from the terminal device 420 registers the information in the database in association with each other.

The analysis device 500 analyzes a sample and generates an analysis result. The analysis device 500 analyzes a sample received from the sample collector A or the storage unit 400. At that time, the analysis device 500 acquires identification information of a container accommodating the sample. The analysis device 500 transmits an analysis result generated by analyzing the sample and the identification information to the server 300. The server 300 that has received the identification information and the analysis result from the analysis device 500 registers the information in the database in association with each other.

The display terminal device 600 receives sample information and/or an analysis result from the database of the server 300 and displays them. The user B requests the server 300 via the display terminal device 600 to disclose sample information and/or an analysis result recorded in the database. The display terminal device 600 acquires corresponding information from the server 300. The display terminal device 600 displays the acquired information on a display unit. The display unit is, for example, an LCD monitor. The display terminal device 600 acquires map information, and displays the sample information together with the map information.

### ≪Registration Contents of Database≫

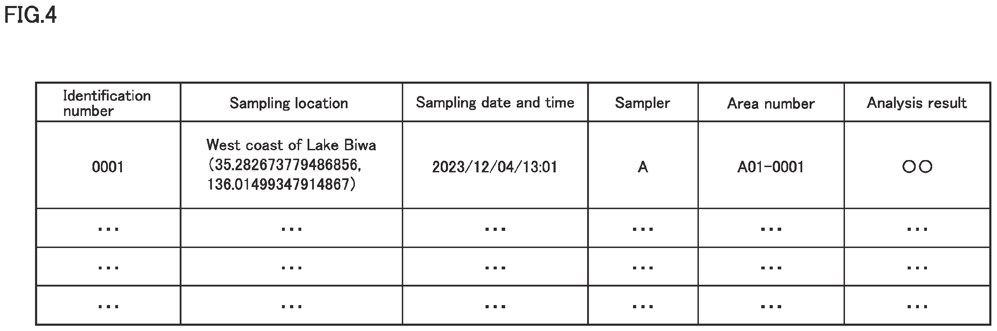

The contents of the database recorded in the server 300 will be described. FIG. 4 is a diagram showing an example of the contents of the database. For example, an identification number of a container, a sampling location of a sample accommodated in the container, a sampling date and time of the sample accommodated in the container, a sampler of the sample accommodated in the container, an area number where the container is stored in the storage 410, and an analysis result of the sample accommodated in the container are recorded in the database. The identification number of a container is an example of identification information, and is a number by which the container can be uniquely distinguished from other containers. The sampling location for the sample is specified based on, for example, an address where a sample was collected inputted by a user, or position information (for example, GPS information) acquired by the terminal device 200. The sample collector is not limited to an individual name, and may be a predetermined personal identification number. The analysis result is a result obtained by analyzing the sample.

### ≪Display Screen≫

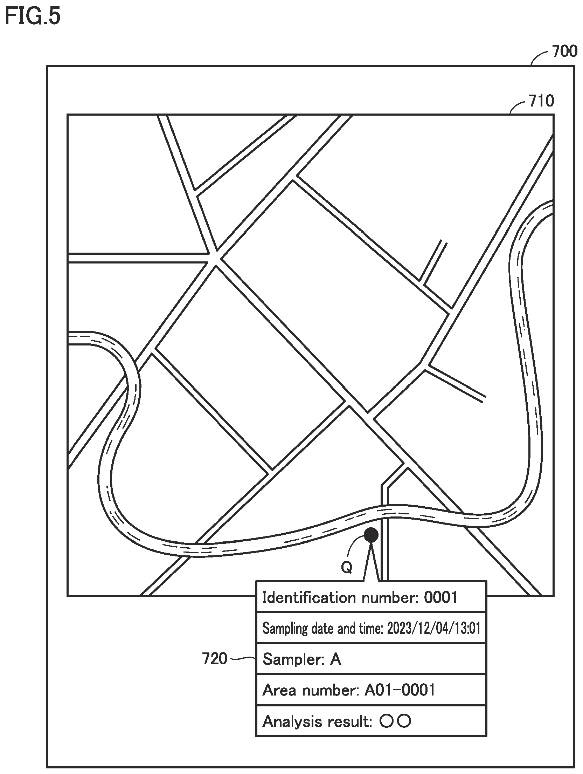

The display terminal device 600 displays sample information and/or an analysis result registered in the database of the server 300 in response to a request from a user B. FIG. 5 is an example of a display screen displayed on the display terminal device 600. A display screen 700 includes a map window 710 and a sample information window 720.

On the map window 710, for example, a map acquired by the display terminal device 600 via the Internet is displayed. Further, in the map window 710, a point Q indicating a position corresponding to a sampling location for the sample registered in the database is displayed.

In the sample information window 720, information of a sample collected at the point Q is displayed.

According to the display screen 700 described above, the user B can easily recognize the location where the sample was collected.

### ≪Flow of Processing in Management System>>

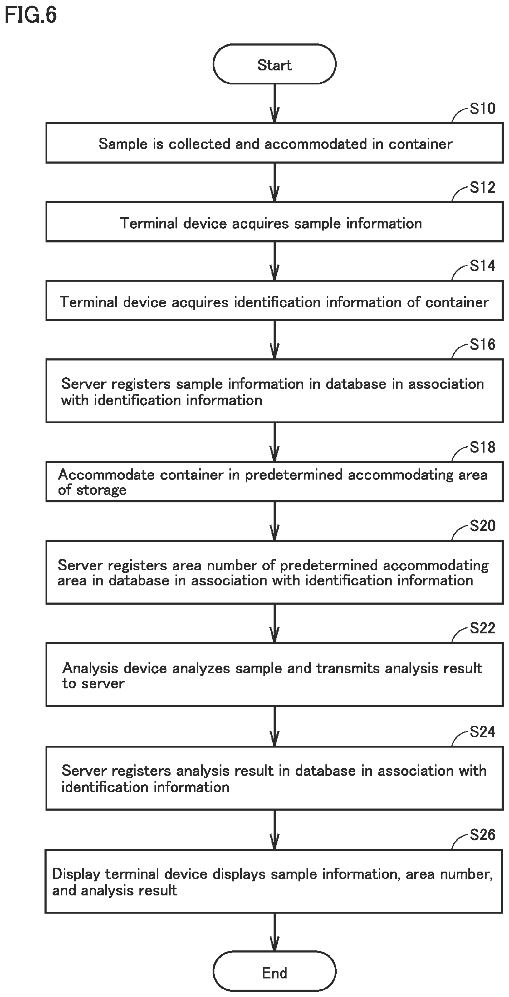

FIG. 6 is a flowchart showing an example of a process in which the management system 1000 manages samples. Of the processing of FIG. 6, step S10 is performed by the sample collector A. Of the processing of FIG. 6, step S12 and step S14 are performed by the terminal device 200. Of the processing of FIG. 6, step S16, step S20, step S22, and step S24 are realized, for example, by the server 300 executing a given program. Of the processing of FIG. 6, step S18 is performed by an operator C who manages containers in the storage unit 400. Of the processing of FIG. 6, step S26 is performed by the display terminal device 600.

Referring to FIG. 6, in step S10, a sample is accommodated in a container by a sample collector A. The container comprises the filter unit 2 and the caps 4. The sample is, for example, a nucleic acid bound to a capture agent. Also, the container may be the storage bag 41 accommodating the capture agent 51 recovered on the filter 72 of the filter unit 7.

In step S12, the terminal device 200 acquires sample information based on an input from the sample collector A via a keyboard or touch panel and/or position information obtained by an antenna.

In step S14, the terminal device 200 acquires identification information of a container accommodating a sample by reading the identifier 22 attached to the container using a scanner or camera possessed by the terminal device 200.

In step S16, the server 300 receives the sample information and the container identification information acquired by the terminal device 200, and registers them in the database in association with each other.

In step S18, an operator C who has received a container accommodating a sample accommodates the container in the storage 410.

In step S20, the operator C causes the terminal device 420 to read the identification information of the container accommodated in step S18. Then, the terminal device 420 transmits the identification information and an area number of an accommodating area in which the container is accommodated to the server 300. The server 300 receives the area number of the accommodating area in which the container is accommodated and the identification information of the container from the terminal device 420, and registers them in the database in association with each other.

In step S22, the analysis device 500 analyzes the sample accommodated in the container, and transmits an analysis result to the server.

In step S24, the server 300 receives the analysis result and the identification information of the container, and registers them in the database in association with each other.

In step S26, the display terminal device 600 displays the sample information, the area number, and the analysis result to the user B.

According to the above-described management system, users can recognize information on samples and position information where the samples are stored, thereby easily finding necessary samples.

According to the above-described management system, users can easily recognize information on samples and analysis results obtained by their use.

### ≪Providing System Using Management System>>

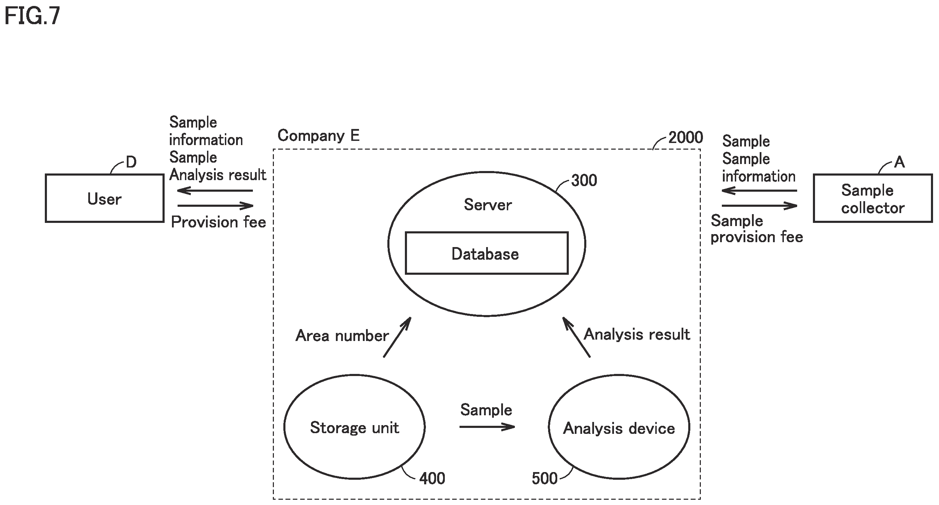

A providing system 2000 that provides samples and/or analysis results together with sample information in response to a request from a user D using the management system 1000 described above will be described. FIG. 7 is a diagram for explaining information and articles transferred in the providing system 2000. The providing system 2000 is managed by a company E that operates the system, and comprises a server 300, a storage unit 400, and an analysis device 500. Note that the analysis device 500 is not an essential element in the providing system 2000 according to the present embodiment, and the company E may acquire analysis results of samples generated by an external analysis device 500.

A sample collector A who collected a sample sends the sample, identification information of a container for the sample, and sample information to the providing system 2000. The server 300 registers the identification information and sample information received from the sample collector A in a database in association with each other. In the providing system 2000, a sample received from the sample collector A is accommodated in a predetermined accommodating area of the storage 410. At that time, an operator C causes the terminal device 420 to read the identification information of the sample container, and transmits the identification information and an area number of the predetermined accommodating area to the server 300. The server 300 registers the identification information and the area number in the database in association with each other. When an analysis device 500 analyzes a sample, an analysis result of the sample is sent to the server 300, and the server 300 registers the analysis result in the database.

The providing system 2000 that has received a sample and its sample information pays a sample provision fee to the sample collector A as consideration for the sample and sample information. The sample provision fee paid from the providing system to the sample collector A is not limited to money, but may be, for example, predetermined service points or the analysis result of the sample. Predetermined service points are privileges on the providing system 2000 that can be used instead of money in the providing system 2000. For example, a person who has received a sample and/or an analysis result from the providing system 2000 can use predetermined service points to pay a provision fee.

A user D acquires sample information, and a sample and/or an analysis result from the providing system 2000. The user D is, for example, a researcher who wants to acquire a sample and/or an analysis result possessed by the providing system 2000 and utilize them for research. The user D receives sample information registered in the database from the providing system 2000. The user D selects a sample to use based on the received sample information, and receives the sample and/or an analysis result of the sample from the providing system 2000. The user D who has received the sample and/or analysis result pays a provision fee to the providing system 2000 as consideration therefor.

The providing system 2000 provides a sample and/or an analysis result of a sample to a user D. When the providing system 2000 provides a sample, the providing system 2000 provides a sample requested by the user D among samples accommodated in the storage 410. The user D who has received the sample can subject the sample to a desired analysis. When the providing system 2000 provides an analysis result of a sample, if the analysis result of a target sample is registered in the database, the providing system 2000 provides the analysis result to the user D. When the analysis result of a target sample is not registered in the database, the target sample is analyzed by the analysis device 500, and the providing system 2000 provides an obtained analysis result to the user D. The providing system 2000 may analyze a sample in advance with the analysis device 500 and register the analysis result in the database, or may analyze a target sample with the analysis device 500 to acquire an analysis result in response to a request from a user D. The providing system 2000 can provide one analysis result to a plurality of users.

### ≪Flow of Processing of Providing System>>

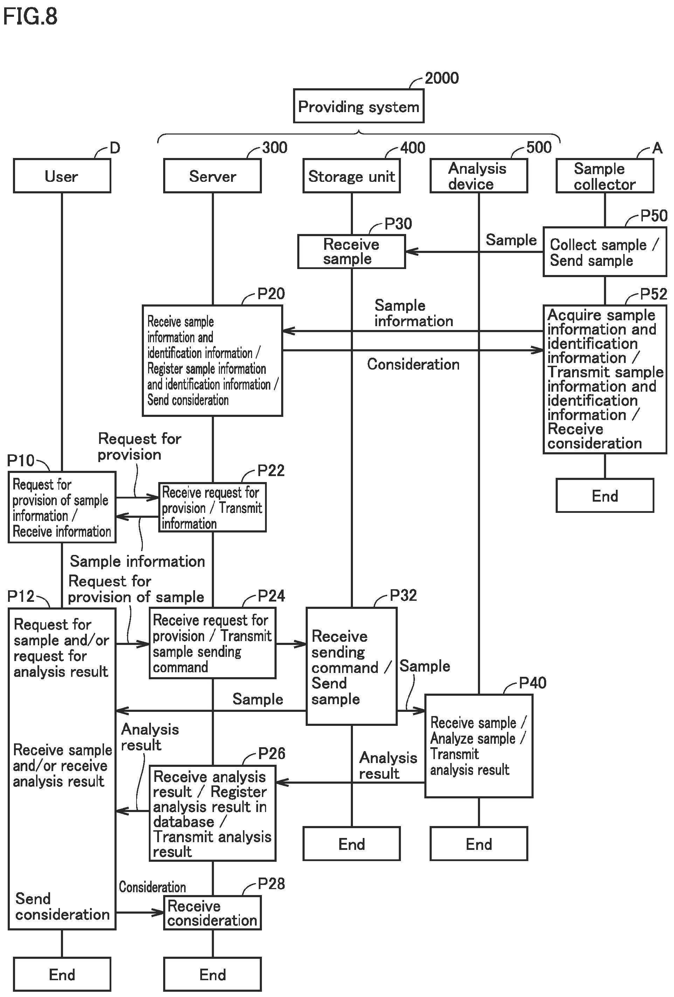

Processing performed among the above-described providing system 2000, sample collector A, and user D will be described. FIG. 8 is a sequence diagram for explaining processes executed in a providing system 2000, a sample collector A, and a user D managed by a company E. Referring to FIG. 8, a sample collector A collects a sample and sends the sample to a company E (P50). Further, the sample collector A uses the terminal device 200 to acquire sample information and identification information of a container for the sample, and transmits them to the server 300 (P52). Then, the sample collector A receives consideration for providing the sample and sample information.

The company E receives a sample from a sample collector A, and an operator of the company E stores the sample in a storage unit of the providing system 2000 (P30). The sample is stored in an accommodating area in the storage 410. When the terminal device 420 of the storage unit 400 receives a command to send a sample from the server 300, an operator of the company E sends the target sample to the user D or the analysis device 500 (P32).

In the providing system 2000, the analysis device 500 analyzes a sample received from the storage 410. The analysis device 500 transmits a generated analysis result to the server 300 (P40).

The server 300 receives sample information from a sample collector A, registers the information in the database, and the company E sends consideration to the sample collector A (P20). Upon receiving a disclosure request for information registered in the database from a user D via the network NW, the server 300 transmits target sample information to the user D (P22). The server 300 receives a request for provision of a sample and/or an analysis result from a user D (P24). When the server 300 determines that it has received a request for sample provision from a user D, it transmits a sample sending command to the terminal device 420 of the storage unit 400 (P24). When the server 300 determines that it has received a request for provision of a sample analysis result from a user D, and the analysis result is not registered in the database, it transmits a sample sending command to the terminal device 420 of the storage unit 400 (P24). When the server 300 determines that it has received a request for provision of a sample analysis result from a user D, and the analysis result is registered in the database, it transmits the analysis result to the user D (P26). Also, the server 300 receives an analysis result from the analysis device 500, registers the analysis result in the database, and transmits the analysis result to the user D (P26). The company E receives consideration for providing the sample and/or analysis result from the user D (P28).

In the user D, a request is made to the server 300 to disclose sample information, and sample information is received from the server 300 (P10). Based on the received sample information, the user D requests a sample and/or an analysis result from the server 300, and after receiving the sample and/or analysis result, sends consideration to the company E (P12).

According to the providing system described above, the company E can obtain profit by receiving consideration from a user D in exchange for a sample and/or analysis result. In addition, the sample collector A can obtain profit by receiving consideration from the company E in exchange for a sample and sample information. Furthermore, by paying consideration, a user D can acquire a sample and/or an analysis result without collecting a sample on site themselves or analyzing a sample themselves.

Heretofore, the method for recovering a nucleic acid, the kit used for the method for recovering a nucleic acid, and the sample management system according to the present embodiment have been described. The method for recovering a nucleic acid of the present embodiment can be used not only for recovering nucleic acids in an environmental liquid sample but also for recovering nucleic acids in a normal liquid sample. That is, the method for recovering a nucleic acid according to the present embodiment may be a method for recovering a nucleic acid present in a liquid sample, comprising:
a preparation step of preparing the liquid sample;
a suspension step of adding a capture agent together with a cationic compound to the prepared liquid sample to generate a suspension; and
a recovery step of recovering the capture agent contained in the suspension,
wherein the capture agent includes a glass fiber or silica, and
the cationic compound has a secondary amino group or a quaternary amino group. The liquid sample includes a liquid sample obtained from a living body (for example, urine, etc.).

### [Examples]

Hereinafter, the present invention will be described in more detail with reference to examples, but the present invention is not limited thereto. In this example, the notation "%" indicates "w/v%" unless otherwise specified.

### «Experiment 1: Preparation of Capture Agent»

A capture agent was prepared in the following procedure. First, to a 10 mL crushing tube, 4 sheets of glass filter paper manufactured by Advantec (product name: GA-55, Φ25 mm) (dry weight: 100 mg), Nuclease-free water (1 mL), and a stainless steel bead (size for 22 mL, 1 piece added) were added. The crushing tube was treated using a Multi-Beads Shocker (manufactured by Yasui Kikai) under conditions of 1500 rpm for 15 seconds to obtain a crushed membrane (powdery glass fiber capture agent). The obtained crushed membrane was used for the following experiments as a crushed membrane for one specimen.

### ≪Experiment 2: Method for recovering nucleic acid present in environmental liquid sample>>

### <Recovery of nucleic acids>

According to the method described in Non-Patent Literature 1, nucleic acids present in environmental liquid samples were recovered by the following procedure.
(1) 400 mL of river water (or 1 L of seawater) was sampled into a 500 mL bottle (or 1 L bottle) (preparation step).
(2) Each additive (a cationic compound such as BAC, flocculant, etc.) was added to the bottle, and well stirred.
(3) The crushed membrane (capture agent) of Experiment 1 was added to the bottle, stirred, and allowed to stand for 1 minute to obtain a suspension (suspension step).
(4) The obtained suspension was filtered by gravity sedimentation using a 40 µm mesh filter. The crushed membrane remaining on the mesh filter was recovered (recovery step).

### <Extraction of nucleic acids>

Nucleic acids were extracted from the recovered crushed membrane by the following procedure.
(1) The recovered crushed membrane was placed in a Salivette tube, ATL buffer (400 µL) and proteinase K (proK) (40 µL) contained in a DNeasy Blood & Tissue kit (manufactured by Qiagen) were further added, and suspended while breaking up the crushed membrane with a pipette tip.
(2) The Salivette tube containing the suspension was incubated at 55°C for 30 minutes.
(3) The Salivette tube was centrifuged under conditions of 3000 g for 3 minutes.
(4) Nuclease-free water (220 µL) was added to the Salivette tube, and left to stand for 1 minute.
(5) The Salivette tube was centrifuged under conditions of 3000 g for 3 minutes.
(6) EtOH (400 µL) was added to the solution at the bottom of the Salivette tube to obtain a mixed liquid.
(7) The mixed liquid (400 µL) was added to a spin column of a DNeasy Blood & Tissue kit (manufactured by Qiagen) and centrifuged under conditions of 6000 g for 1 minute.
(8) After discarding the flow-through, the remaining mixed liquid was added, and centrifuged under conditions of 6000 g for 1 minute.
(9) After discarding the flow-through, WB1 (500 µl) contained in the kit was added to the spin column, and centrifuged under conditions of 6000 g for 1 minute.
(10) After discarding the flow-through, WB2 (500 µl) contained in the kit was added to the spin column, and centrifuged under conditions of 6000 g for 1 minute.
(11) The filter was transferred to a new tube, and centrifuged at maximum speed (19,000 g) of a centrifuge for 2 minutes.
(12) The filter was taken out without coming into contact with the wall of the tube, and set in a DNA LoBind tube (Eppendorf).
(13) AE buffer (100 µL) was added to the tube in which the filter was set, and left to stand for 1 minute.
(14) The tube was centrifuged under conditions of 6000 g for 1 minute.
(15) The concentration of a nucleic acid in the solution at the bottom of the tube was measured. Thereafter, the tube containing the solution was stored at -80°C.

The concentration of nucleic acid was measured by properly using nucleic acid quantification by Nanodrop and nucleic acid quantification by Qubit. When determining the average value, the average value was calculated with N=3.

Nucleic acid quantification by Nanodrop: The nucleic acid concentration was quantified from the absorbance using 2 µL of purified nucleic acid in a Nanodrop ND-1000. Nucleic acid quantification by Qubit: The nucleic acid concentration was quantified using a nucleic acid quantification kit (manufactured by Thermo Fisher, product names: 1× dsDNA HS and BR RNA).

### ≪Experiment 3: Influence of benzalkonium chloride on nucleic acid yield>>

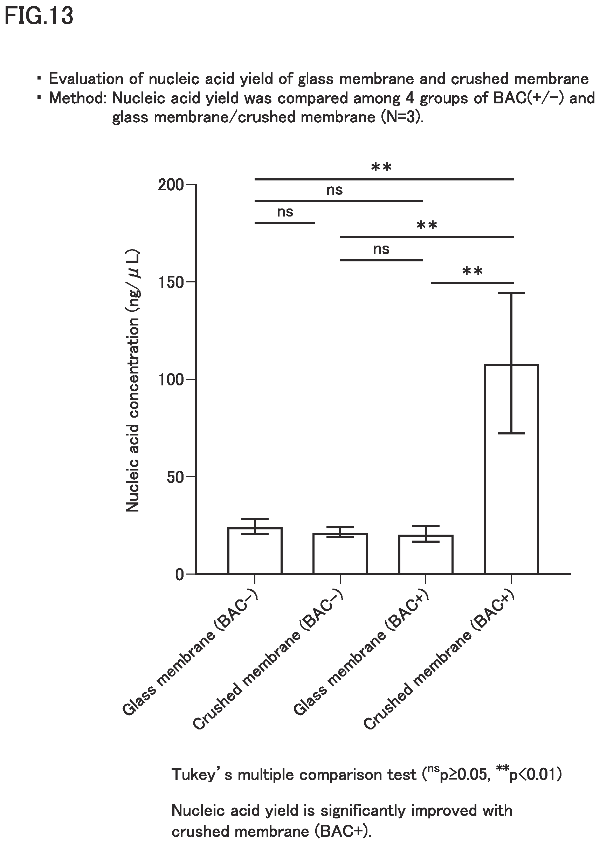

The change in nucleic acid yield was examined by adding benzalkonium chloride (cationic compound, hereinafter sometimes abbreviated as "BAC") manufactured by Fujifilm Wako to an environmental liquid sample. As a capture agent, a crushed membrane prepared by the method described above (corresponding to the Example) and glass filter papers before crushing (4 sheets, corresponding to the Comparative Example) were prepared. For a method of recovering nucleic acid, the same method as in Experiment 2 was used. At this time, a group to which BAC was added (BAC+, corresponding to an Example, final concentration 0.01%) and a group to which BAC was not added (BAC-, corresponding to a Comparative Example) were provided. River water (400 mL) was used as an environmental liquid sample. The quantification of nucleic acid was performed by Nanodrop. The results are shown in FIG. 13.

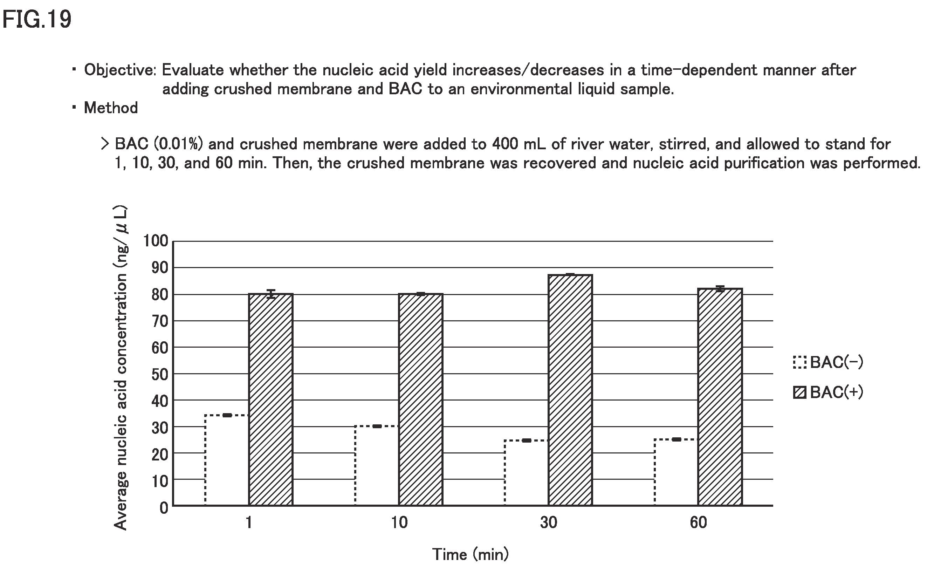

From the results of FIG. 13, it was found that the yield of nucleic acid was significantly improved when the crushed membrane and BAC were added to an environmental liquid sample (crushed membrane (BAC+)). Although it was conventionally known to add BAC to an environmental liquid sample for long-term preservation, and to add a crushed membrane to an environmental liquid sample to recover nucleic acids, it was not known that the yield of nucleic acid was significantly improved when both were used in combination, which is a finding that the present inventors have discovered for the first time. Normally, degradation of nucleic acids is limited in a short time. For example, in Experiment 21 described later, nucleic acids in environmental liquid samples were hardly degraded within at least 1 hour (see BAC(-) in FIG. 19). Also, in Experiment 26 described later, nucleic acids in TBS buffer were hardly degraded within at least 2 hours (see left figure in FIG. 24). Considering these points, the present inventors consider that a cationic compound such as BAC contributes to an improvement in binding between a crushed membrane and a nucleic acid (for example, FIG. 9).

### ≪Experiment 4: Examination of the order of addition of BAC>>

Whether there was an influence on nucleic acid yield when changing the order of adding a crushed membrane and BAC to an environmental liquid sample was examined. The same experiment as Experiment 3 was conducted, except that the order of addition was changed. The quantification of nucleic acid was performed by Nanodrop. The results are shown in Table 1.

**[Table 1]**

| Group configuration (In parentheses: Order of adding BAC) | River water | BAC (final 0.01%) | Crushed membrane (100mg) | Average nucleic acid concentration (ng/µL±SD) |
|---|---|---|---|---|
| Crushed membrane (-) | 400mL | - | + | 16.3±0.87 |
| Crushed membrane (BAC → Crushed membrane) | 400mL | + | + | 56.2±0.82 |
| Crushed membrane (Crushed membrane → BAC) | 400mL | + | + | 72.3±0.79 |

From the results in Table 1, it was found that regardless of the order of adding the capture agent and the cationic compound (BAC), the nucleic acid yield was improved in groups to which the cationic compound was added compared to a group to which the cationic compound was not added.

### ≪Experiment 5-1: Examination of ratio of crushed membrane to BAC>>

Using 400 mL of river water, the mass ratio of crushed membrane amount / BAC amount (crushed membrane amount / promoter amount ratio) was examined. The same experiment as in Experiment 3 was conducted except that crushed membranes and BAC were used in ratios shown in Table 2. The quantification of nucleic acid was performed by Nanodrop. The results are shown in Table 3A.

**[Table 2]**

| Crushed membrane/Promoter amount ratio | | Promoter amount (mg) | | | |
|---|---|---|---|---|---|
| | | 0 | 0.4 | 4 | 40 |
| Crushed membrane amount (mg) | 10 | - | 25 | 2.5 | 0.25 |
| | 25 | - | 62.5 | 6.25 | 0.625 |
| | 50 | - | 125 | 12.5 | 1.25 |
| | 100 | - | 250 | 25 | 2.5 |

**[Table 3A]**

| Average nucleic acid concentration (ng/µL±SD) | | BAC amount (mg) | | | |
|---|---|---|---|---|---|
| | | 0 | 0.4 | 4 | 40 |
| Crushed membrane amount (mg) | 10 | 44.8±0.25 | 55.1±0.76 | 80.9±0.63 | 50.5±0.56 |
| | 25 | 83.1±1.58 | 99.6±0.76 | 110.2±1.14 | 76.2±0.83 |
| | 50 | 83.6±0.33 | 88.4±0.7 | 126.9±0.74 | 124.3±1.08 |
| | 100 | 32±0.78 | 40.5±0.6 | 43.9±0.66 | 92.1±1.02 |

From the results in Tables 2 and 3A, when comparing those having the same amount of crushed membrane, there was a tendency for nucleic acid yield to be higher in groups to which BAC was added than in groups to which BAC was not added. Further, it was confirmed that the yield of nucleic acid was improved when the crushed membrane amount/BAC amount ratio was in a range of 0.25 to 250. Furthermore, it was confirmed that the yield of nucleic acid was further improved when the crushed membrane amount/BAC amount ratio was in a range of 1.25 to 62.5.

### «Experiment 5-2: Examination of ratio of crushed membrane to domiphen bromide>>

Using 400 mL of river water, the mass ratio of crushed membrane amount / domiphen bromide amount (crushed membrane amount / promoter amount ratio) was examined. The same experiment as in Experiment 3 was conducted except that crushed membranes and domiphen bromide were used in the ratios shown in Table 2 above. The quantification of nucleic acid was performed by Nanodrop. The results are shown in Table 3B.

**[Table 3B]**

| Average nucleic acid concentration (ng/µL±SD) | | Domiphen bromide amount (mg) | | | |
|---|---|---|---|---|---|
| | | 0 | 0.4 | 4 | 40 |
| Crushed membrane amount (mg) | 10 | 22.7±0.14 | 32.8±0.73 | 45.3±1.18 | 24.4±1.32 |
| | 25 | 24.6±0.97 | 44±1.24 | 62.3±1.75 | 50.5±1.28 |
| | 50 | 35.6±0.25 | 56.5±1.76 | 47.7±1.25 | 54.9±0.97 |
| | 100 | 14.2±0.52 | 14.1±0.45 | 19.9±0.87 | 65.4±0.58 |

From the results in Tables 2 and 3B, when comparing those having the same amount of crushed membrane, there was a tendency for nucleic acid yield to be higher in groups to which domiphen bromide was added than in groups to which domiphen bromide was not added. In addition, it was confirmed that the yield of nucleic acid was improved when the crushed membrane amount/domiphen bromide amount ratio was in a range of 0.25 to 250. Furthermore, it was confirmed that the yield of nucleic acid was further improved when the crushed membrane amount/domiphen bromide amount ratio was in a range of 0.625 to 125.

### «Experiment 6: Examination of BAC concentration>>

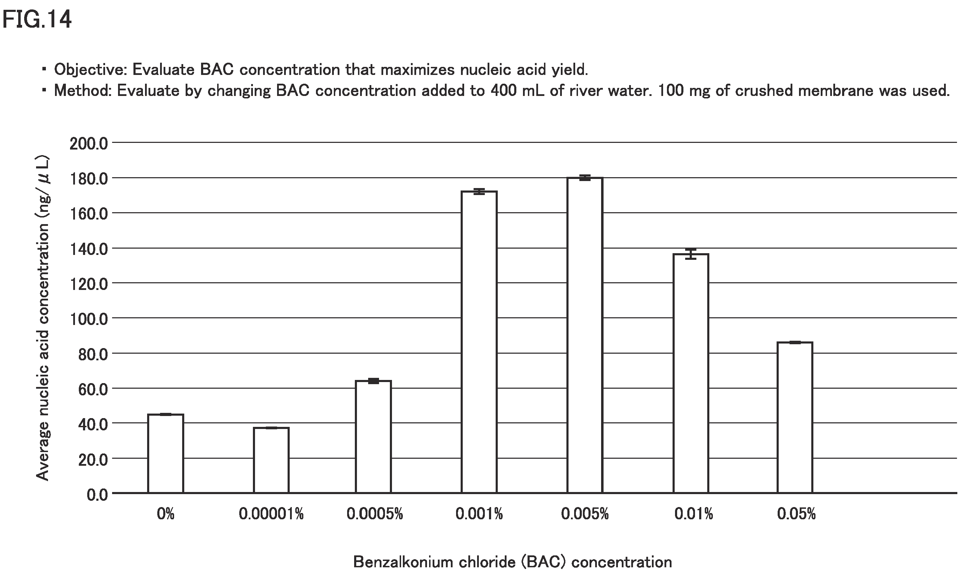

The change in nucleic acid yield was evaluated by changing the concentration of BAC added to 400 mL of river water. 100 mg of a crushed membrane was used (equivalent to 4 sheets of glass filter paper). The same experiment as Experiment 3 was conducted except that the concentration (final concentration) of BAC was changed. The quantification of nucleic acid was performed by Nanodrop. The results are shown in FIG. 14.

From the results of FIG. 14, it was found that the nucleic acid yield was maximized when the concentration of BAC was in a range of 0.001% to 0.01%.

### «Experiment 7: Examination of concentration of other cationic compounds>>

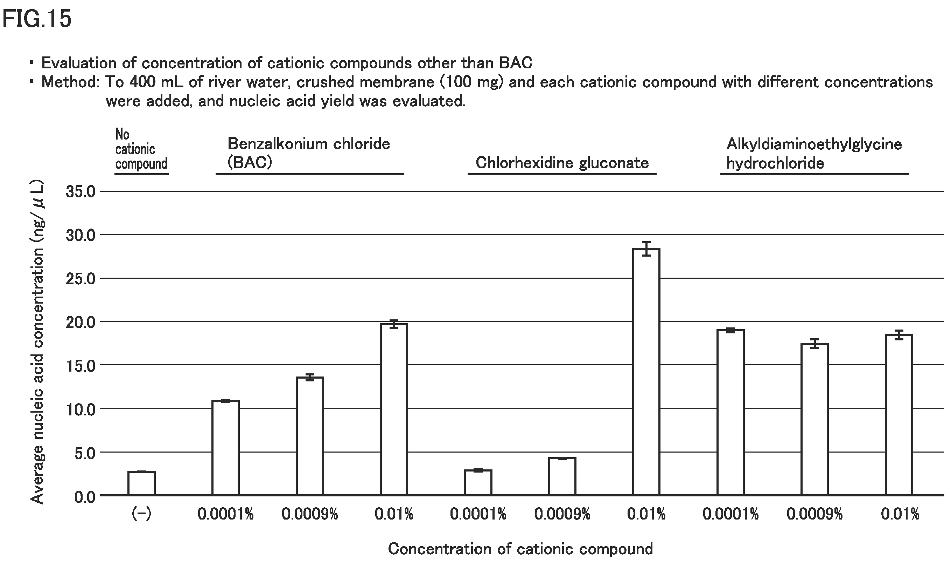

Changes in nucleic acid yield were evaluated by varying the concentration of cationic compounds other than BAC. 100 mg of a crushed membrane was used (equivalent to 4 sheets of glass filter paper). As other cationic compounds, chlorhexidine gluconate (manufactured by Fujifilm Wako) and alkyldiaminoethylglycine hydrochloride (manufactured by Fujifilm Wako) were used. The same experiment as Experiment 3 was conducted except that the concentration (final concentration) of the cationic compound was changed. The quantification of nucleic acid was performed by Nanodrop. The results are shown in FIG. 15.

From the results in FIG. 15, it was found that not only BAC, which is a compound having a quaternary amino group (quaternary ammonium salt), but also chlorhexidine gluconate and alkyldiaminoethylglycine hydrochloride, which are compounds having a secondary amino group (secondary ammonium salts), contribute to an improvement in nucleic acid yield. In addition, it was found that chlorhexidine gluconate and alkyldiaminoethylglycine hydrochloride are effective in improving nucleic acid yield in a concentration range comparable to that of BAC.

### «Experiment 8: Examination of other capture agents>>

Whether nucleic acid yield could be improved using glass filter papers other than the glass filter paper manufactured by Advantec (product name: GA-55, Φ25 mm) and silica filter papers was examined. Using the glass filter papers or silica filter papers shown in Table 4, crushed membranes of each were prepared in the same method as in Experiment 1.

**[Table 4]**

| Product Name | Type | Size (Φ) (mm) | Number of Sheets | Mass (mg) |
|---|---|---|---|---|
| GA-55 | Glass filter paper | 25 | 4 | 100 |
| GA-100 | Glass filter paper | 25 | 2 | 110 |
| GC-50 | Glass filter paper | 25 | 4 | 95 |
| GS-25 | Glass filter paper | 25 | 3 | 103 |
| QR-100 | Silica filter paper | 24 | 3 | 115 |

The same experiment as in Experiment 3 was conducted except that each of the prepared crushed membranes was used. The quantification of nucleic acid was performed by Nanodrop. The results are shown in Table 5.

**[Table 5]**

| Group configuration | River water | BAC | Crushed membrane (Product name) | Average nucleic acid concentration (ng/µL) | SD |
|---|---|---|---|---|---|
| GA-55 (-BAC) | 400mL | - | GA-55 | 25.8 | 0.46 |
| GA-100 (-BAC) | 400mL | - | GA-100 | 8.4 | 1.01 |
| GC-50 (-BAC) | 400mL | - | GC-50 | 30.0 | 0.24 |
| GS-25 (-BAC) | 400mL | - | GS-25 | 16.4 | 0.45 |
| QR-100 (-BAC) | 400mL | - | QR-100 | 28.8 | 0.90 |
| GA-55+BAC | 400mL | + | GA-55 | 89.4 | 0.97 |
| GA-100+BAC | 400mL | + | GA-100 | 75.9 | 0.45 |
| GC-50+BAC | 400mL | + | GC-50 | 92.8 | 0.42 |
| GS-25+BAC | 400mL | + | GS-25 | 80.6 | 1.52 |
| QR-100+BAC | 400mL | + | QR-100 | 102.8 | 0.93 |

From the results in Table 5, it was found that the yield of nucleic acid was significantly improved by using a crushed membrane obtained from any glass filter paper or silica filter paper in combination with BAC.

### «Experiment 9-1: Examination when using glass powder as a capture agent>>

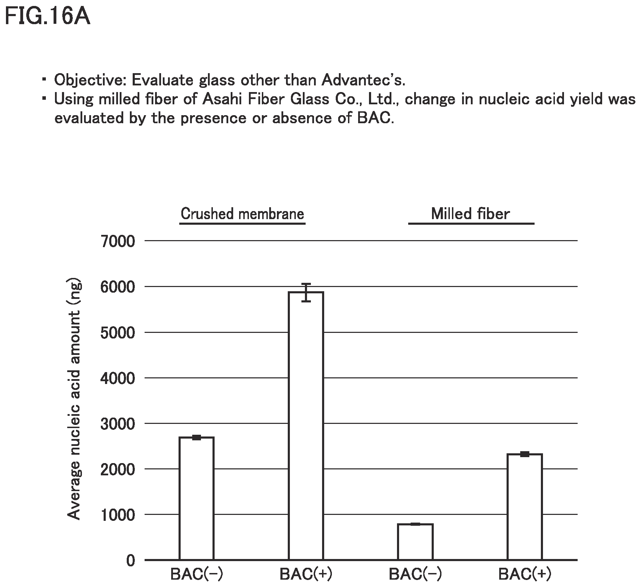

Instead of Advantec glass filter paper, a milled fiber manufactured by Asahi Fiber Glass Co., Ltd. (product name: MF20JH1-20, glass fiber length crushed to a length of 100 to 300 µm) was used as a capture agent to examine nucleic acid yield. The same experiment as Experiment 3 was conducted except that the milled fiber (150 mg) was used as a capture agent. The quantification of nucleic acid was performed by Qubit. The results are shown in FIG. 16A.

From the results in FIG. 16A, it was found that even when the above milled fiber is used as a capture agent, the nucleic acid yield is improved by using it in combination with BAC.

### «Experiment 9-2: Examination when using glass powder as a capture agent»

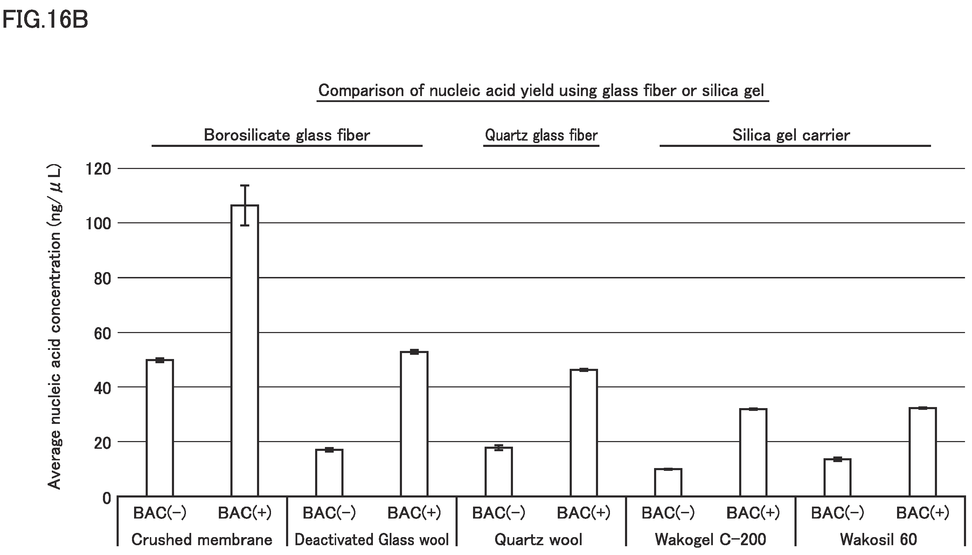

Instead of Advantec glass filter paper, glass fibers or silica gel (silica carrier) shown in Table 6 below were used as capture agents to examine nucleic acid yield. The same experiment as Experiment 3 was conducted except that the glass fibers or silica gel were used as a capture agent under conditions shown in Table 6 below. The quantification of nucleic acid was performed by Nanodrop. The results are shown in FIG. 16B.

**[Table 6]**

| Name | Material | Present/Absent of crushing | Amount used | Model number, Manufacturer |
|---|---|---|---|---|
| Crushed membrane | Borosilicate glass fiber | Present | 100mg | 36261025, Advantec Toyo |
| Deactivated Glass wool | Borosilicate glass fiber | Present | 200mg | #20789, Restek |
| Quartz wool | Quartz glass fiber | Present | 200mg | 6-570-01, AS ONE |
| Wakogel C-200 | Silica gel | Absent | 200mg | 237-00075, wako |
| Wakosil 60, 64-210µm | Silica gel | Absent | 200mg | 230-02765, wako |

From the results in FIG. 16B, it was found that even when the above glass fiber or silica gel is used as a capture agent, the yield of nucleic acid is improved by using it in combination with BAC.

### «Experiment 10: Examination of other surfactants>>

BAC is a type of cationic surfactant. Whether nucleic acid yield would be improved when using other surfactants (anionic surfactants, nonionic surfactants) was examined. The same experiment as in Experiment 3 was conducted except that each component shown in Table 6 was used instead of BAC. The quantification of nucleic acid was performed by Nanodrop. The results are shown in Table 7.

**[Table 7]**

| Component name | River water (mL) | Final concentration of each component (%) | Characteristic of each component | Average nucleic acid concentration (ng/µL±SD) |
|---|---|---|---|---|
| Control | 400 | - | - | 84.2±0.7 |
| Benzalkonium chloride | 400 | 0.001 | Cation | 444.6±3.7 |
| Benzalkonium chloride | 400 | 0.01 | Cation | 313.2±3.3 |
| Benzalkonium chloride | 400 | 0.1 | Cation | 211.6±1.7 |
| Chlorhexidine gluconate | 400 | 0.01 | Cation | 289.6±2.1 |
| Alkyldiaminoethylglycine hydrochloride | 400 | 0.01 | Amphoteric | 412.3±2.4 |
| Tween20 | 400 | 0.01 | Nonionic | 78.0±0.9 |
| Lithium dodecyl sulfate | 400 | 0.01 | Anion | 102.2±1.2 |

From the results of Table 7, it was found that nucleic acid yields were significantly improved with cationic compounds such as BAC, chlorhexidine gluconate, and alkyldiaminoethylglycine hydrochloride. On the other hand, lithium dodecyl sulfate, an anionic surfactant, resulted in only a slight increase in nucleic acid yield. Furthermore, the nonionic Tween20 showed almost no change in nucleic acid yield.

### «Experiment 11: Examination of chaotropic salts>>

In general, a chaotropic salt is used to improve yield when binding a nucleic acid to a silica carrier. Therefore, how chaotropic salts affect nucleic acid yield when a nucleic acid is recovered using the capture agent in this example was examined. The same experiment as Experiment 3 was conducted except that guanidine thiocyanate (chaotropic salt) was used instead of BAC. The quantification of nucleic acid was performed by Nanodrop. The results are shown in Table 8.

**[Table 8]**

| Group configuration | River water | BAC (0.001%) | Guanidine thiocyanate (3M) | Average nucleic acid concentration (ng/µL±SD) |
|---|---|---|---|---|
| Crushed membrane only | 400mL | - | - | 30.6±0.51 |
| Crushed membrane + BAC | 400mL | + | - | 90.7±0.17 |
| Crushed membrane + Guanidine thiocyanate | 400mL | - | + | 32.3±0.33 |

From the results of Table 8, even when guanidine thiocyanate, which is regarded as highly effective as a chaotropic salt, was used, an improvement in nucleic acid yield could not be confirmed. This result suggested that the improvement in nucleic acid yield achieved by using a capture agent in combination with BAC is due to a mechanism (e.g., FIG. 9) different from chaotropic salts conventionally used for nucleic acid purification with a spin column.

### «Experiment 12: Examination of pH>>

Whether the pH at the time of nucleic acid recovery affects nucleic acid yield was examined. The same experiment as Experiment 3 was conducted except that 200 mL of collected river water was adjusted to pH 5.3, 7.2, or 9.3 with a 6 mol/L hydrochloric acid solution (Fujifilm Wako Pure Chemical) and a 5 mol/L sodium hydroxide solution (Fujifilm Wako Pure Chemical), and BAC or chlorhexidine gluconate was used as a cationic compound. The quantification of nucleic acid was performed by Nanodrop. The results are shown in Table 9.

**[Table 9]**

| Group configuration | pH after adjustment | Crushed membrane (33mg) | BAC (0.001%) | Chlorhexidine gluconate (0.01%) | Average nucleic acid concentration (ng/µL±SD) |
|---|---|---|---|---|---|
| Control pH5.3 | 5.3 | + | - | - | 30.4±0.82 |
| Control pH7.2 | 7.2 | + | - | - | 29.7±0.29 |
| Control pH9.3 | 9.3 | + | - | - | 39.6±1.33 |
| BAC pH5.3 | 5.3 | + | + | - | 60.8±0.31 |
| BAC pH7.2 | 7.2 | + | + | - | 59.3±0.95 |
| BAC pH9.3 | 9.3 | + | + | - | 57.0±0.36 |
| Chlorhexidine gluconate pH5.3 | 5.3 | + | - | + | 47.5±0.26 |
| Chlorhexidine gluconate pH7.2 | 7.2 | + | - | + | 50.2±0.69 |
| Chlorhexidine gluconate pH9.3 | 9.3 | + | - | + | 57.9±0.66 |

From the results of Table 9, it was found that at any pH, the yield of nucleic acid was improved by using a capture agent and a cationic compound in combination.

### «Experiment 13: Nucleic acid spike experiment>>

Impurity materials such as cells and bacteria are present in environmental liquid samples, but whether the effect is exhibited when using BAC and a capture agent in combination even for a liquid sample without such impurities was examined. Specifically, a purified nucleic acid (5000 ng) was first added (spiked) into 400 mL of TBS (20 mM Tris-HCl + 150 mM NaCl) to prepare a liquid sample. Using the prepared liquid sample, the same experiment as in Experiment 3 was conducted except that it was conducted under the conditions shown in Table 10. For example, in the experiment indicated by Sample No. 1 in Table 10, the liquid sample was first passed through a glass membrane (glass filter paper) in the absence of BAC (BAC(-)), and the amount of nucleic acid recovered on the glass membrane was quantified. At this time, the liquid sample passing through the glass membrane (flow-through) was brought into contact with a crushed membrane in the presence of BAC (BAC(+)) to recover and quantify the nucleic acid again. The same applies to experiments indicated by other numbers. The quantification of nucleic acid was performed by Qubit. The results are shown in Table 10.

**[Table 10]**

| Sample No. | Group configuration (In parentheses: BAC:+/-) | Added solution | BAC (0.001%) | Amount of added nucleic acid (ng) | Crushed membrane | Average nucleic acid amount (ng) | Average recovery rate |
|---|---|---|---|---|---|---|---|
| 1 | Glass membrane (-) | 1×TBS 400mL | - | 5000 | - | 5.6±0.17 | 0.11% |
| | Glass membrane (-) → Crushed membrane(+) | Flow-through 400mL | + | - | + | 1177±9.43 | 24% |
| 2 | Crushed membrane(-) | 1×TBS 400mL | - | 5000 | + | 6.2±0.06 | 0.12% |
| | Crushed membrane(-) → Crushed membrane(+) | Flow-through 400mL | + | - | + | 1117±4.71 | 22% |
| 3 | Glass membrane (+) | 1×TBS 400mL | + | 5000 | - | 52.4±0.28 | 1.0% |
| | Glass membrane (+) → Crushed membrane(+) | Flow-through 400mL | - | - | + | 193.7±0.47 | 3.9% |
| 4 | Crushed membrane(+) | 1×TBS 400mL | + | 5000 | + | 1123±4.71 | 22% |
| | Crushed membrane(+) → Crushed membrane(+) | Flow-through 400mL | - | - | + | 2.4±0.02 | 0.061% |

From the results of Table 10, the following was found. In BAC(-), the nucleic acid recovery rates of both the glass membrane and the crushed membrane were approximately 0.1% and unchanged, but it was possible to recover nucleic acids when the flow-through was concentrated by a crushed membrane (BAC+) (Sample Nos. 1, 2). In BAC(+), the recovery rate increased even with a glass membrane, but it was around 1% (Sample No. 3). On the other hand, the crushed membrane improved the recovery rate up to 22% (Sample No. 4). As for flow-throughs, for the crushed membrane (BAC+) following the glass membrane treatment, it slipped into the flow-through at 3.9% (Sample No. 3). On the other hand, from the flow-through following the crushed membrane (BAC+), the nucleic acid recovery rate was approximately 0.06%, indicating that a large amount of nucleic acid was successfully recovered by the crushed membrane (BAC+) (Sample No. 4). The nucleic acid recovery rate of 22% for the crushed membrane (BAC+) implies that the remainder may be (A) incapable of dissociating from the capture agent at the time of extraction, or (B) capable of dissociating from the capture agent, but lost because it cannot be adsorbed on the column of the kit.

### «Experiment 14: Evaluation of fiber length of crushed membrane>>

The fiber length of a crushed membrane was evaluated according to the following procedure. First, each glass filter paper manufactured by Advantec shown in Table 11 was added to a 10 mL tube, and for those subjected to wet crushing, water (1 mL) was added, and crushing was performed with a stainless steel bead at 1500 rpm for 5 to 30 seconds. For the crushing, a Multi-Beads Shocker (manufactured by Yasui Kikai) was used. The obtained crushed membranes were measured with Valmet FS5 (manufactured by Valmet) to determine the fiber length. The results are shown in Table 11. From the results in Table 11, it was found that the arithmetic average fiber length of the crushed membrane (glass fiber) is 100 µm to 300 µm.

**[Table 11]**

| Fiber Analysis | Membrane Type | GA-55 | | | | GA-100 | GC-50 | GS-25 | QR-100 | |
|---|---|---|---|---|---|---|---|---|---|---|
| | Crushing time (sec) | 5 | 15 | 30 | 15 | 15 | 15 | 15 | 15 | 30 |
| | Wet crushing | + | + | + | - | + | + | + | + | + |
| Weight average fiber length | mm | 0.76 | 0.61 | 0.57 | 0.34 | 0.89 | 0.67 | 1.09 | 0.3 | 0.71 |
| Arithmetic average fiber length | mm | 0.19 | 0.18 | 0.18 | 0.16 | 0.27 | 0.17 | 0.2 | 0.16 | 0.15 |
| Length average fiber length | mm | 0.32 | 0.28 | 0.28 | 0.21 | 0.48 | 0.26 | 0.34 | 0.2 | 0.24 |
| Average fiber width | µm | 15.32 | 16.42 | 16.42 | 13.59 | 12.97 | 15.14 | 31.99 | 13.1 | 16.72 |

### «Experiment 15: Examination of flocculant (river water)>>

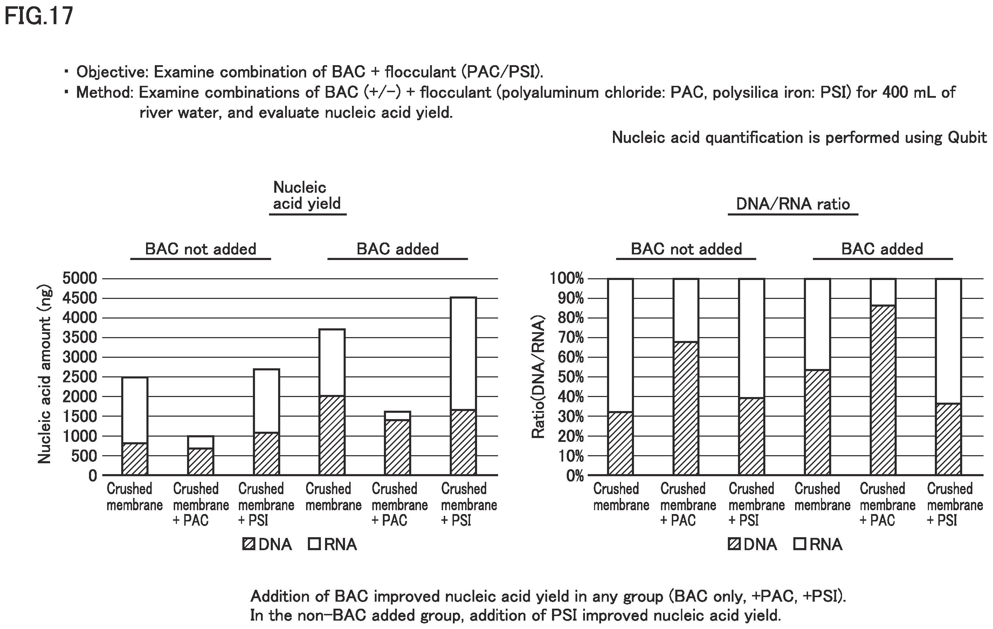

The nucleic acid yield when using a flocculant in addition to a capture agent and a cationic compound was examined. For river water (400 mL), combinations of BAC (+/-) and flocculants (polyaluminum chloride: PAC, polysilica iron: PSI) were examined, and the nucleic acid yield was evaluated. At this time, the final concentrations of PAC and PSI were 0.005 v/v% and 0.0125 v/v%, respectively. The same experiment as Experiment 3 was conducted except that a flocculant was newly used. The quantification of nucleic acid was performed by Qubit. The results are shown in FIG. 17. From the results in FIG. 17, it was found that the addition of BAC improved the nucleic acid yield in any group (BAC only, BAC+PAC, BAC+PSI). In the non-BAC added group, the addition of PSI improved the nucleic acid yield.

### «Experiment 16: Examination of polymer flocculant (river water)>>

The nucleic acid yield when using a polymer flocculant was examined. For river water (400 mL), each combination of no promoter, BAC only, BAC+PSI, BAC+Fe₃O₄+PSI, and BAC+PSI+polymer flocculant (N-131 or A-102T) was examined to evaluate nucleic acid yield. The same experiment as Experiment 3 was conducted except that the above combinations were used. The quantification of nucleic acid was performed by Qubit. The results are shown in Table 12 and FIGS. 10 to 12.

**[Table 12]**

| Group configuration (In parentheses: BAC+/-) | River water | BAC (0.001%) | Crushed membrane (100mg) | Fe₃O₄ (0.02g) | PSI (50µL) | Polymer flocculant (0.0001%) | Average nucleic acid amount (ng±SD) |
|---|---|---|---|---|---|---|---|
| Crushed membrane | 400mL | - | + | - | - | - | 2,718±21 |
| Crushed membrane+PSI | 400mL | - | + | - | + | - | 13,560±221 |
| Crushed membrane+Fe₃O₄ +PSI | 400mL | - | + | + | + | - | 6,477±215 |
| Crushed membrane | 400mL | + | + | - | - | - | 5,853±176 |
| Crushed membrane+PSI | 400mL | + | + | - | + | - | 18,133±466 |
| Crushed membrane+Fe₃O₄ +PSI | 400mL | + | + | + | + | - | 20,387±523 |
| Crushed membrane+PSI +(N-131)^{*} | 400mL | + | + | - | + | + (N-131) | 17,060±589 |
| Crushed membrane+PSI +(A-102T)^{**} | 400mL | + | + | - | + | + (A-102T) | 12,353±759 |

| | | | | | | | |
|---|---|---|---|---|---|---|---|
| *N-131: Nonionic, molecular weight 1600 **A-102T: Weakly anionic, molecular weight 1700 | | | | | | | |

From the results of Table 12, it was found that the yield of nucleic acid was also improved by combining flocculants (PSI, PAC, polymer flocculant) in addition to a capture agent and a cationic compound. Further, from the photographs in FIGS. 10 and 11, it was found that the capture agent could float in an agglomerated state by combining and using flocculants. From the photograph in FIG. 12, it was found that by using a flocculant and triiron tetraoxide, the capture agent can be recovered with a magnet in an agglomerated state.

### «Experiment 17: Examination of flocculant (seawater)>>

The same experiment as Experiment 16 was conducted except that seawater (1000 mL) was used instead of river water (400 mL). The quantification of nucleic acid was performed by Nanodrop. The results are shown in Table 13.

**[Table 13]**

| Group configuration (In parentheses: BAC +/-) | Seawater | BAC (0.001%) | Crushed membrane (100mg) | PSI (50µL) | Polymer flocculant (A-102T, 400µL) | Nucleic acid concentration (ng/µL) |
|---|---|---|---|---|---|---|
| Glass membrane (-) | 1000mL | - | - | - | - | 28 |
| Crushed membrane(-) | 1000mL | - | + | - | - | 140 |
| Crushed membrane+PSI(-) | 1000mL | - | + | + | - | 397 |
| Glass membrane (+) | 1000mL | + | - | - | - | 28 |
| Crushed membrane(+) | 1000mL | + | + | - | - | 336 |
| Crushed membrane+PSI(+) | 1000mL | + | + | + | - | 439 |
| Crushed membrane+PSI +Polymer flocculant (102T)(+) | 1000mL | + | + | + | + | 304 |

From the results of Table 13, it was found that even in BAC(-), the yield of nucleic acid was improved by adding a flocculant (PSI). Further, in the BAC(+) group, it was found that the nucleic acid yield was improved except for the case where a glass membrane was used. Especially in the combination of BAC+PSI, the yield of nucleic acid was improved.

### «Experiment 18: Evaluation of silica carrier>>

Whether nucleic acid yield would be improved even when a silica carrier was used as a capture agent was examined. The same experiment as Experiment 3 was conducted except that silica carriers and a crushed membrane shown below were used as capture agents. The quantification of nucleic acid was performed by Nanodrop. The results are shown in Table 14.
Used silica carriers (all manufactured by EVONIK)
CARPLEX #80: General-purpose crushed product (average particle size 15 µm)
CARPLEX BS-306: Gel type product (average particle size 24 µm)
CARPLEX BS-510BX: Gel type product (average particle size 10.5 µm)

**[Table 14]**

| Group configuration (In parentheses: BAC+/-) | BAC | Crushed membrane | Silica | Nucleic acid concentration (ng/µL) |
|---|---|---|---|---|
| Crushed membrane(-) | - | 100mg | - | 22 |
| Crushed membrane+#80(-) | - | 100mg | #80 50mg | 24 |
| Crushed membrane+BS-306(-) | - | 100mg | BS-306 50mg | 12.6 |
| BS-306(-) | - | - | BS-306 100mg | 4.7 |
| Crushed membrane+510BX(-) | - | 100mg | 510BX 50mg | 15.2 |
| Crushed membrane(+) | + | 100mg | - | 96.8 |
| Crushed membrane+#80(+) | + | 100mg | #80 50mg | 50.3 |
| Crushed membrane+BS-306(+) | + | 100mg | BS-306 50mg | 48.9 |
| BS-306(+) | + | - | BS-306 100mg | 33.6 |
| Crushed membrane+510BX(+) | + | 100mg | 510BX 50mg | 49.6 |

From the results in Table 14, it was found that even when a silica carrier was used as a capture agent, the nucleic acid yield was improved by using it in combination with BAC. In particular, when CARPLEX BS-306 was used as a capture agent, the nucleic acid yield was improved approximately 7-fold.

### «Experiment 19: Evaluation of diversity of recovered nucleic acids>>

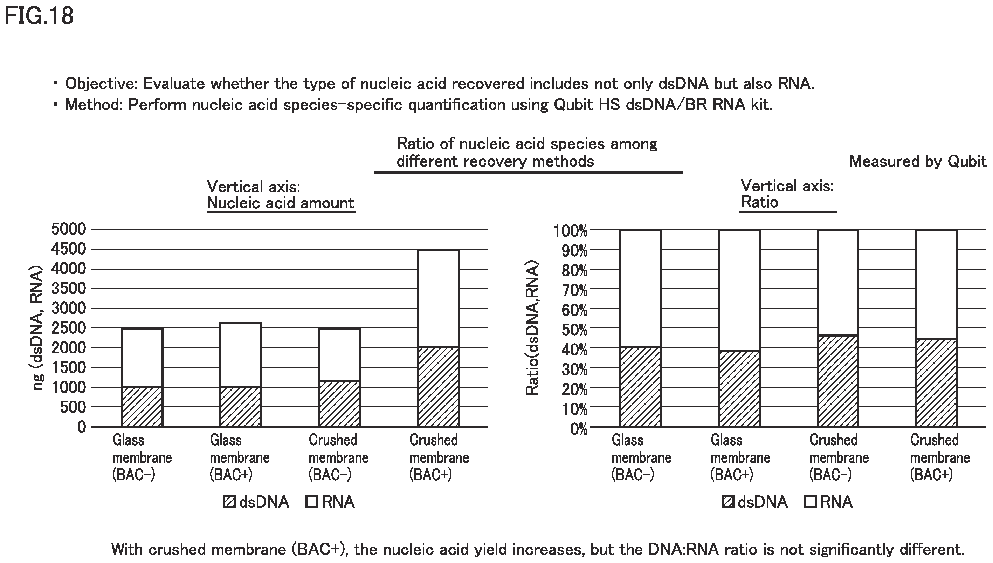

Whether the recovered nucleic acids contain not only dsDNA but also RNA was evaluated. The same experiment as Experiment 3 was conducted except that river water was used as an environmental liquid sample and a glass filter paper and a crushed membrane were used as capture agents. dsDNA and RNA were each quantified by nucleic acid quantification with Qubit. The results are shown in FIG. 18.

From the results in FIG. 18, it was found that when a crushed membrane and BAC are used in combination, the nucleic acid yield increases, but the DNA:RNA ratio is not significantly different compared to when a crushed membrane alone is used.

### «Experiment 20: Examination of other cationic compounds (1)>>

Whether nucleic acid yield would be improved when a cationic compound other than the cationic compounds used in the experiments thus far was used was examined. The same experiment as Experiment 3 was conducted except that cationic compounds shown in Table 15 were used. The quantification of nucleic acid was performed by Qubit. The results are shown in Table 15. As for the cationic compounds listed in Table 15, domiphen bromide from Sigma-Aldrich was used. As for other cationic compounds, products manufactured by Fujifilm Wako Pure Chemical Corporation were used.

**[Table 15]**

| Group configuration | River water | Final concentration of cationic compound | Crushed membrane (100mg) | Average nucleic acid concentration (ng/µL±SD) |
|---|---|---|---|---|
| Control | 400mL | - | + | 2.8±0.034 |
| Benzalkonium chloride(BAC) | 400mL | 0.001% | + | 14.7±0.26 |
| 1-Decyl-3-methylimidazolium chloride | 400mL | 0.001% | + | 3.5±0081. |
| 1-Dodecylpyridinium chloride | 400mL | 0.001% | + | 5.8±0.11 |
| Domiphen bromide | 400mL | 0.001% | + | 13.1±0.12 |
| Hexadecyltrimethylammonium bromide | 400mL | 0.001% | + | 10.4±0.16 |

From the results of Table 15, it was found that any of the cationic compounds improves nucleic acid yield by being used in combination with a capture agent. Particularly, domiphen bromide and hexadecyltrimethylammonium bromide exhibited effects comparable to BAC.

### «Experiment 21: Examination of standing time after adding a crushed membrane, etc.>>

Whether nucleic acid yield changes by changing the standing time after adding a crushed membrane and a cationic compound to an environmental liquid sample was examined. The same experiment as Experiment 3 was conducted except that BAC (final concentration 0.01%) and a crushed membrane were added to river water (400 mL), stirred, and then the standing times were set to 1 minute, 10 minutes, 30 minutes, and 60 minutes, respectively. The quantification of nucleic acid was performed by Nanodrop. The results are shown in FIG. 19.

From the results in FIG. 19, it was found that there is no significant change in the yield of nucleic acid even if the standing time is changed. In other words, it was found that the method for recovering a nucleic acid according to the present example can be processed in a short time by setting the standing time to 1 minute. Moreover, from the results of BAC(-), it was suggested that nucleic acids in environmental liquid samples hardly degrade within at least 1 hour.

### «Experiment 22: Examination of nucleic acid recovery from sewage>>

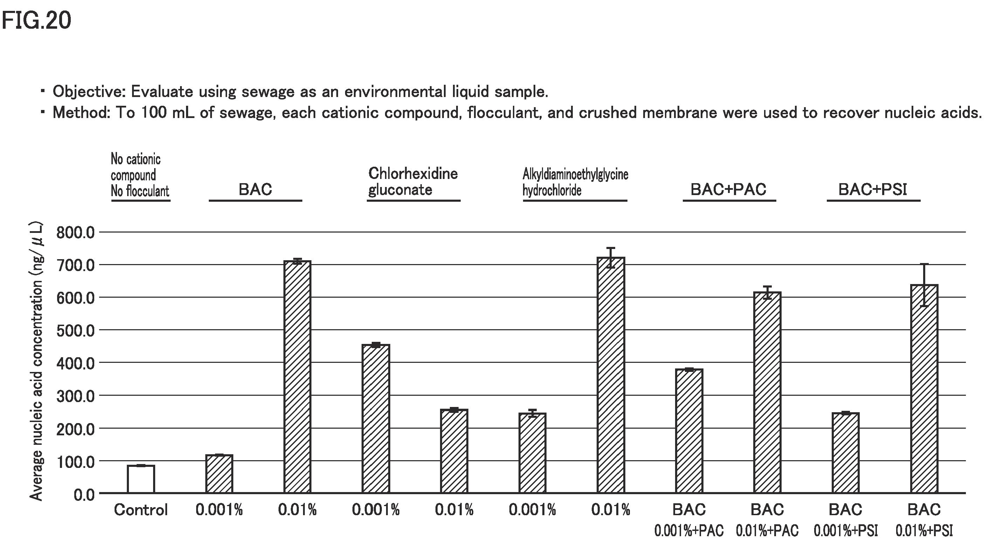

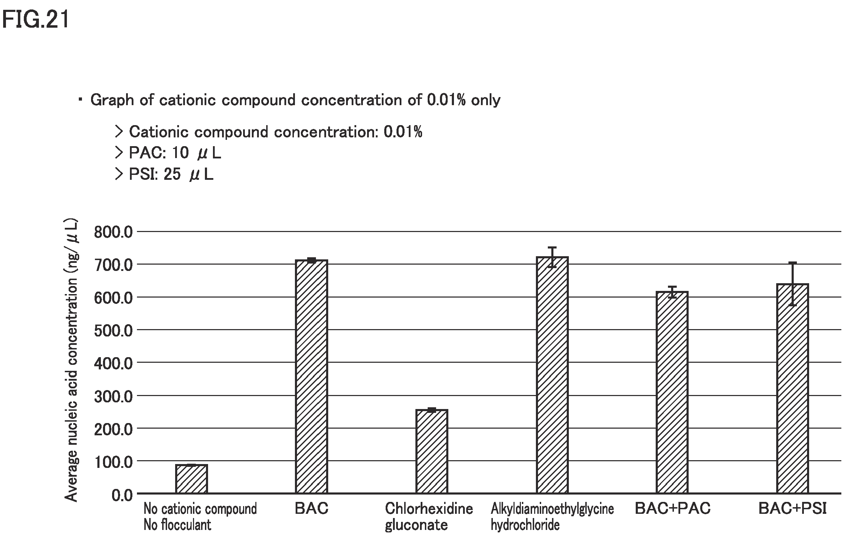

When sewage is used as an environmental liquid sample, it was examined whether the yield of nucleic acid is improved. The same experiment as Experiment 3 was conducted except that sewage (100 mL) was used as the environmental liquid sample, BAC, chlorhexidine gluconate, and alkyldiaminoethylglycine hydrochloride were used as cationic compounds, and PAC and PSI were used as flocculants. The quantification of nucleic acid was performed by Nanodrop. The results are shown in FIGS. 20 and 21. From the results of FIGS. 20 and 21, it was found that the yield of nucleic acid was improved even when sewage was used as an environmental liquid sample.

### «Experiment 23: Pre-mixing of capture agent and cationic compound>>

In the procedure of Experiment 2, a capture agent and a cationic compound are separately added to an environmental liquid sample. Whether the yield of nucleic acid would change even if the capture agent and the cationic compound were pre-mixed into a composition (mixture) and the composition was then added to an environmental liquid sample was examined. The recovery method using the composition (sample marked as "pre-mixing" in Table 16) was performed by the following procedure.
(1) In a sample container, a crushed membrane and BAC were mixed to obtain a composition (1 h).
(2) An environmental liquid sample was added to the sample container containing the composition, stirred, and allowed to stand for 1 minute.
(3) The capture agent was recovered by a filter, and nucleic acids were extracted and quantified in the same manner as in Experiment 2. The quantification of nucleic acid was performed by Nanodrop.

**[Table 16]**

| Group configuration (In parentheses: BAC +/-) | River water | BAC | Timing of BAC addition | Crushed membrane | Average nucleic acid concentration (ng/µL) | SD |
|---|---|---|---|---|---|---|
| Crushed membrane(-) | 400mL | - | - | 100mg | 45.4 | 0.125 |
| Crushed membrane(BAC 0.001%) | 400mL | + | Directly added to environmental sample | 100mg | 171.7 | 1.156 |
| Crushed membrane(BAC 0.01%) | 400mL | + | Directly added to environmental sample | 100mg | 136.3 | 2.538 |
| Crushed membrane (BAC 0.001%, Pre-mixing) | 400mL | + | Pre-mixed with crushed membrane in sample container | 100mg | 64.7 | 0.283 |
| Crushed membrane (BAC 0.01%, Pre-mixing) | 400mL | + | Pre-mixed with crushed membrane in sample container | 100mg | 184.6 | 1.190 |

The results are shown in Table 16. Samples in Table 16 without the note of "Pre-mixing" were subjected to the same experiment as in Experiment 3 to recover nucleic acids. From the results in Table 16, it was found that even when a capture agent and a cationic compound were pre-mixed and an environmental liquid sample was added thereto, the yield of nucleic acids improved in the optimum concentration ranges (0.001%, 0.01%) relative to the control experiment (capture agent without addition of BAC).

### «Experiment 24: Examination of surface area of capture agent»

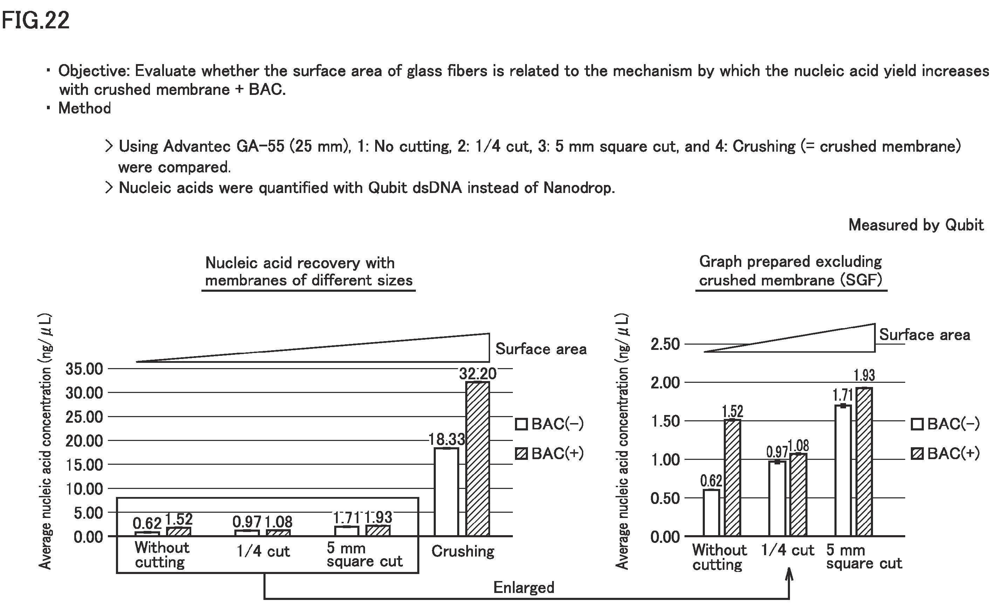

Whether there is a change in the yield of nucleic acid by changing the surface area of a capture agent was examined. The same experiment as Experiment 3 was conducted except that the following four types were used as capture agents, and nucleic acids were quantified by Qubit dsDNA. The quantification of nucleic acid was performed by Qubit. The results are shown in FIG. 22.
Used capture agents (all prepared from Advantec GA-55 (25 mm) filter paper)
Glass filter paper (without cutting)
Glass filter paper cut into 1/4
Glass filter paper cut into 5 mm square
Crushed membrane crushed by the method of Experiment 1

From the results in FIG. 22, it was observed that for the same amount of glass filter paper, the nucleic acid yield tended to improve as the size became smaller (as the surface area increased). In addition, in any capture agent, the nucleic acid yield was improved by adding BAC.

### «Experiment 25: Examination of other cationic compounds (2)>>

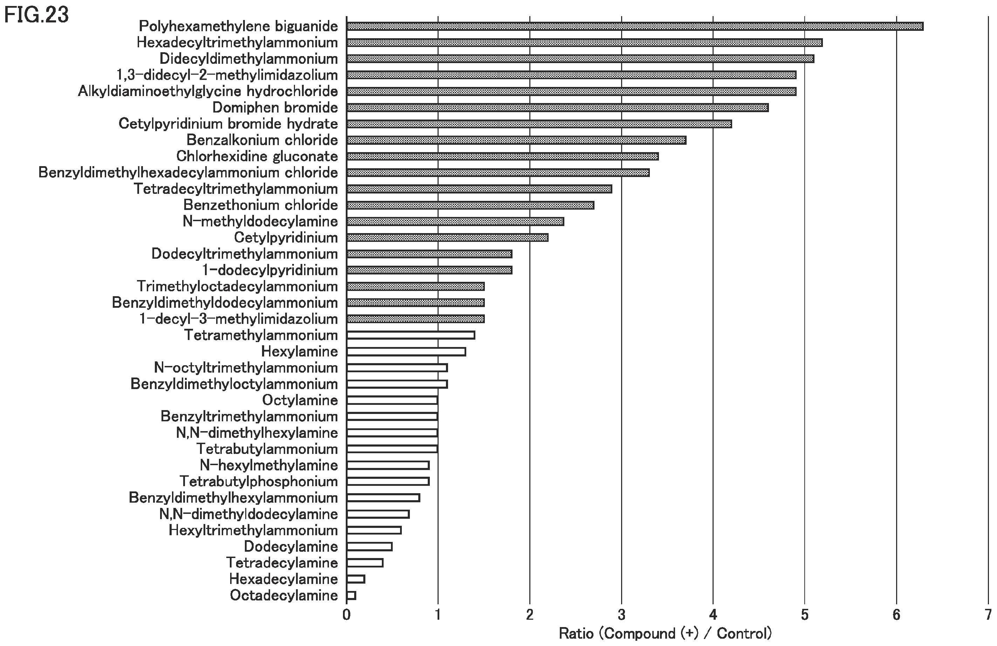

Whether nucleic acid yield would be improved when a cationic compound other than the cationic compounds used in the experiments thus far was used was examined. The same experiment as Experiment 3 was conducted except that cationic compounds shown below were used, and the final concentration of the cationic compound was set to 0.01%. The quantification of nucleic acid was performed by Nanodrop. The recovery ratio was calculated based on the recovery amount of nucleic acid in a control experiment (control) performed without using a cationic compound. The results are shown in FIG. 23. In FIG. 23, the vertical axis indicates the cationic compound used (final concentration 0.01%), and the horizontal axis indicates the nucleic acid recovery ratio based on the control. Note that, due to space limitations in the description column, names of cationic compounds are partially omitted in FIG. 23.

### (List of cationic compounds used)

Polyhexamethylene biguanide hydrochloride (Fujifilm Wako Pure Chemical Corporation), hexadecyltrimethylammonium bromide (Fujifilm Wako Pure Chemical Corporation), 1,3-didecyl-2-methylimidazolium chloride (Sigma-Aldrich), alkyldiaminoethylglycine hydrochloride (Fujifilm Wako Pure Chemical Corporation), domiphen bromide (Sigma-Aldrich), benzalkonium chloride (Fujifilm Wako Pure Chemical Corporation), chlorhexidine gluconate (Fujifilm Wako Pure Chemical Corporation), tetradecyltrimethylammonium chloride (Fujifilm Wako Pure Chemical Corporation), N-methyldodecylamine (Sigma-Aldrich), cetylpyridinium (Fujifilm Wako Pure Chemical Corporation), 1-dodecylpyridinium chloride (Fujifilm Wako Pure Chemical Corporation), dodecyltrimethylammonium chloride (Sigma-Aldrich), trimethyloctadecylammonium chloride (Sigma-Aldrich), benzyldimethyldodecylammonium chloride (Sigma-Aldrich), 1-decyl-3-methylimidazolium chloride (Fujifilm Wako Pure Chemical Corporation), tetramethylammonium chloride (Sigma-Aldrich), hexylamine (Sigma-Aldrich), benzyldimethyloctylammonium chloride (Sigma-Aldrich), benzyltrimethylammonium hydroxide (Fujifilm Wako Pure Chemical Corporation), N,N-dimethylhexylamine (Sigma-Aldrich), tetrabutylammonium chloride (Sigma-Aldrich), N-hexylmethylamine (Sigma-Aldrich), tetrabutylphosphonium chloride (Sigma-Aldrich), benzyldimethylhexylammonium chloride (Sigma-Aldrich), N,N-dimethyldodecylamine (Sigma-Aldrich), dodecylamine (Sigma-Aldrich), tetradecylamine (Sigma-Aldrich), hexadecylamine (Sigma-Aldrich), octadecylamine (Sigma-Aldrich), hexyltrimethylammonium (Sigma-Aldrich), benzyldimethylhexylammonium (Sigma-Aldrich), octylamine (Fujifilm Wako Pure Chemical Corporation), N-octyltrimethylammonium (Sigma-Aldrich), benzyldimethylhexadecylammonium chloride (Sigma-Aldrich), cetylpyridinium bromide hydrate (Sigma-Aldrich), didecyldimethylammonium (Fujifilm Wako Pure Chemical Corporation).

From the results in FIG. 23, it was found that polyhexamethylene biguanide, hexadecyltrimethylammonium bromide, 1,3-didecyl-2-methylimidazolium, alkyldiaminoethylglycine hydrochloride, domiphen bromide, benzalkonium chloride, chlorhexidine gluconate, tetradecyltrimethylammonium, N-methyldodecylamine, cetylpyridinium, 1-dodecylpyridinium chloride, dodecyltrimethylammonium, trimethyloctadecylammonium, benzyldimethyldodecylammonium, 1-decyl-3-methylimidazolium chloride, benzyldimethylhexadecylammonium chloride, cetylpyridinium bromide, and didecyldimethylammonium improved the nucleic acid yield by 1.5 times or more compared to the control experiment when used in combination with a capture agent.

### «Experiment 26: Stability of nucleic acid in TBS buffer>>

The stability of a nucleic acid was examined by varying the standing time after adding the nucleic acid to a TBS buffer. Specifically, first, 500 ng of a nucleic acid was added to a 1× TBS buffer (20 mM Tris-HCl + 150 mM NaCl) (1 mL). The concentration of the nucleic acid after 0 to 120 minutes had elapsed since the addition was quantified using Qubit. The results are shown in FIG. 24 (left figure). From the results of the left figure in FIG. 24, it was found that the nucleic acid in the TBS buffer did not show a large concentration change and was stable without degradation for up to 120 minutes after addition.

In addition, a recovery amount when nucleic acids in a TBS buffer were recovered in the same manner as in Experiment 3 was examined. Specifically, 5000 ng of a nucleic acid was added to a 1× TBS buffer (400 mL). Further, the same experiment as Experiment 3 was conducted except that BAC (final concentration 0% or 0.01%) and a crushed membrane were added and stirred, and then standing times were set to 1 minute, 5 minutes, 30 minutes, and 60 minutes, respectively. The quantification of nucleic acid was performed by Qubit. The results are shown in FIG. 24 (right figure). From the results of the right figure in FIG. 24, it was found that there is no large change in the yield of nucleic acid even if the above standing time is changed. In other words, it was found that the method for recovering a nucleic acid according to the present example can be processed in a short time by setting the standing time to 1 minute.

### «Experiment 27: Examination of nucleic acid recovery from clinical specimen>>

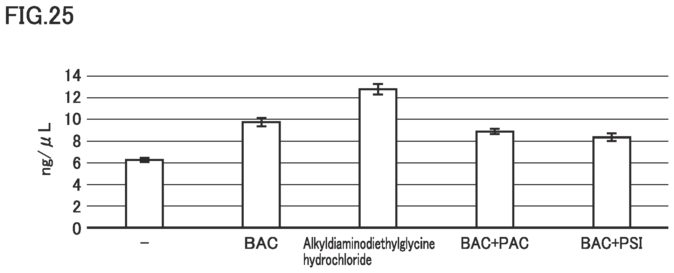

When a clinical specimen (urine) was used as a liquid sample, it was examined whether the yield of nucleic acid improved. The same experiment as Experiment 3 was conducted except that a human urine pool specimen (100 mL) was used as a liquid sample, BAC and alkyldiaminoethylglycine hydrochloride were used as cationic compounds, and PAC (10 µL) and PSI (25 µL) were used as flocculants. The quantification of nucleic acid was performed by Qubit. The results are shown in FIG. 25. From the results in FIG. 25, it was found that the yield of nucleic acid was improved even when a clinical specimen (urine) was used as a liquid sample.

### «Experiment 28: Examination of sheet-form capture agent>>

Whether nucleic acid yield would be improved when a sheet-form capture agent was used was examined. A sheet-form capture agent was prepared in the following procedure. First, to a 10 mL crushing tube, 4 sheets of glass filter paper manufactured by Advantec (product name: GA-55, Φ25 mm) (dry weight: 100 mg), Nuclease-free water (1 mL), and a stainless steel bead (size for 22 mL, 1 piece added) were added. The crushing tube was treated using a Multi-Beads Shocker (manufactured by Yasui Kikai) under conditions of 1500 rpm for 15 seconds to obtain a crushed membrane (powdery glass fiber capture agent). The obtained crushed membrane was dried at 40°C for 3 days and formed into a sheet-form capture agent. The same experiment as Experiment 3 was conducted except that the obtained sheet-form capture agent was used as a capture agent for one specimen. The results are shown in Table 17. The crushed membrane in Table 17 is a crushed membrane prepared according to Experiment 1.

**[Table 17]**

| Group configuration (In parentheses: BAC addition) | BAC (Final 0.01%) | Capture agent (100mg) | Average nucleic acid yield (ng/µL) ±Standard Deviation |
|---|---|---|---|
| Crushed membrane(-) | - | Crushed membrane | 28.5±0.33 |
| Crushed membrane(+) | + | Crushed membrane | 172.3±2.7 |
| Sheet-form capture agent (-) | - | Sheet-form capture agent | 40.5±0.29 |
| Sheet-form capture agent (+) | + | Sheet-form capture agent | 186.1±3.18 |

From the results in Table 17, it was found that the yield of nucleic acid was improved even when a sheet-form capture agent was used.

### [Aspects]

It will be understood by those skilled in the art that the plurality of exemplary embodiments and examples described above are specific examples of the following aspects.

### (Item 1)

A method for recovering a nucleic acid according to one aspect is a method for recovering a nucleic acid present in an environmental liquid sample, comprising:
a preparation step of preparing the environmental liquid sample;
a suspension step of adding a capture agent together with a cationic compound to the prepared environmental liquid sample to generate a suspension; and
a recovery step of recovering the capture agent contained in the suspension,
wherein the capture agent includes a glass fiber or a silica carrier, and
the cationic compound has a secondary amino group or a quaternary amino group.

According to the method for recovering a nucleic acid of Item 1, a method for recovering a nucleic acid with an improved yield can be provided.

### (Item 2)

In the method for recovering a nucleic acid of Item 1, the capture agent includes a powdery glass fiber. According to the method for recovering a nucleic acid of Item 2, the nucleic acid yield is further improved.

### (Item 3)

In the method for recovering a nucleic acid of Item 2, an arithmetic average fiber length of the powdery glass fiber is 20 µm or more and 500 µm or less. According to the method for recovering a nucleic acid of Item 3, the nucleic acid yield is further improved.

### (Item 4)

In the method for recovering a nucleic acid according to any one of Items 1 to 3, a material of the glass fiber includes borosilicate glass.

### (Item 5)

In the method for recovering a nucleic acid according to any one of Items 1 to 4, the cationic compound further has a straight-chain alkyl group having 10 to 18 carbon atoms. According to the method for recovering a nucleic acid of Item 5, the nucleic acid yield is further improved.

### (Item 6)

In the method for recovering a nucleic acid according to any one of Items 1 to 5, the cationic compound includes a compound having a quaternary amino group and having a straight-chain alkyl group having 10 or more carbon atoms, or a compound having a secondary amino group and having a bactericidal activity. According to the method for recovering a nucleic acid of Item 6, the nucleic acid yield is further improved.

### (Item 7)

In the method for recovering a nucleic acid according to any one of Items 1 to 6, the cationic compound includes at least one selected from the group consisting of benzalkonium chloride, chlorhexidine gluconate, alkyldiaminoethylglycine hydrochloride, polyhexamethylene biguanide, N-methyldodecylamine, domiphen bromide, hexadecyltrimethylammonium bromide, 1-decyl-3-methylimidazolium chloride, 1,3-didecyl-2-methylimidazolium, tetradecyltrimethylammonium, cetylpyridinium, dodecyltrimethylammonium, trimethyloctadecylammonium, benzyldimethyldodecylammonium, 1-dodecylpyridinium chloride, benzyldimethylhexadecylammonium chloride, cetylpyridinium bromide, and didecyldimethylammonium. According to the method for recovering a nucleic acid of Item 7, the nucleic acid yield is further improved.

### (Item 8)

In the method for recovering a nucleic acid according to Item 7, the cationic compound includes benzalkonium chloride, and a concentration of the benzalkonium chloride is 0.0005 w/v% or more and 0.05 w/v% or less based on the suspension. According to the method for recovering a nucleic acid of Item 8, the nucleic acid yield is further improved.

### (Item 9)

In the method for recovering a nucleic acid according to any one of Items 1 to 8, a mass ratio of the capture agent to the cationic compound in the suspension is 0.25 or more and 250 or less. According to the method for recovering a nucleic acid of Item 9, the nucleic acid yield is further improved.

### (Item 10)

In the method for recovering a nucleic acid according to any one of Items 1 to 9, in the suspension step, the cationic compound is added in order to promote binding between the capture agent and the nucleic acid. According to the method for recovering a nucleic acid of Item 10, the cationic compound is added for the purpose of promoting binding between the capture agent and the nucleic acid.

### (Item 11)

In the method for recovering a nucleic acid according to any one of Items 1 to 10, the method further includes adding a flocculant in the suspension step, and the flocculant includes at least one selected from the group consisting of polyaluminum chloride, polysilica iron compounds, and polymer flocculants. According to the method for recovering a nucleic acid of Item 11, recovery of the capture agent adsorbing the nucleic acid is facilitated.

### (Item 12)

In the method for recovering a nucleic acid according to Item 11, the method further includes adding triiron tetraoxide in the suspension step. According to the method for recovering a nucleic acid of Item 12, by applying a magnetic force, the capture agent can be collected at a predetermined position.

### (Item 13)

In the method for recovering a nucleic acid according to any one of Items 1 to 12, the environmental liquid sample includes at least one selected from the group consisting of river water, seawater, lake water, and sewage. The method for recovering a nucleic acid according to Item 13 is suitably used for river water, seawater, lake water, sewage, and the like.

### (Item 14)

In the method for recovering a nucleic acid according to any one of Items 1 to 13, in the recovery step, the capture agent is recovered by filtering the suspension. According to the method for recovering a nucleic acid of Item 14, recovery of the capture agent adsorbing the nucleic acid is facilitated.

### (Item 15)

In the method for recovering a nucleic acid according to any one of Items 1 to 14, in the preparation step, the environmental liquid sample does not contain the cationic compound.

### (Item 16)

A kit for recovering a nucleic acid in one aspect is a kit for recovering a nucleic acid comprising a composition used for recovering a nucleic acid contained in a liquid sample, wherein the composition comprises:
a capture agent including a glass fiber or a silica carrier; and
a cationic compound having a secondary amino group or a quaternary amino group.

According to the recovery kit of Item 16, recovery efficiency of a nucleic acid contained in a liquid sample can be improved.

### (Item 17)

A kit for recovering a nucleic acid according to one aspect comprises:
a sampling bag having an opening;
a filter unit engaging with the opening and having a filter; and
a cap configured to form a cavity with the filter by engaging with the filter unit. According to the recovery kit of Item 17, a burden on a user required to recover nucleic acids contained in a liquid sample can be reduced.

### (Item 18)

The recovery kit according to Item 16 may further include the sampling bag, the filter unit, and the cap according to Item 17. According to the recovery kit of Item 18, articles necessary to carry out the method for recovering a nucleic acid are provided.

### (Item 19)

The filter unit included in the recovery kit according to Item 17 or Item 18 may have an identifier indicating identification information attached thereto. According to the recovery kit of Item 19, recovered nucleic acids can be identified based on identification information.

### (Item 20)

A management system according to one aspect is a management system that manages a sample accommodated in a container to which an identifier indicating identification information is attached and information of the sample,
comprising a terminal device, a server, and a storage configured to be capable of mutually communicating information via a network,
wherein the storage is capable of accommodating the container,
the terminal device includes an acquisition unit that acquires the information of the sample and the identification information, and
the server stores the identification information and the information of the sample in association with each other. According to the management system of Item 20, the server stores a container with an identifier attached and information of a sample accommodated in the container in association with each other. Therefore, a user can easily acquire information of a sample accommodated in the container based on the sample container.

### (Item 21)

In the management system according to Item 20, the sample may be at least one of river water, sewage, seawater, and a biological sample. According to the management system of Item 21, a sample that is at least one of river water, sewage, seawater, and a biological sample can be managed together with information of the sample.

### (Item 22)

In the management system according to Item 20 or Item 21, the acquisition unit has a receiver for acquiring position information,
the information of the sample includes a location where the sample was collected, and
the location where the sample was collected is specified by the position information acquired by the receiver. According to the management system of Item 22, information on a location where a sample specified by position information received by a terminal device was collected can be managed together with identification information.

### (Item 23)

The management system according to any one of Items 20 to 22 further comprises an analysis device,
wherein the analysis device analyzes the sample to acquire an analysis result, and
the server stores the analysis result in association with the identification information. According to the management system of Item 23, a user can easily acquire information on a sample and an analysis result of the sample based on identification information.

### (Item 24)

The management system according to any one of Items 20 to 23 further comprises a display terminal device,
wherein the display terminal device displays the information of the sample. According to the management system of Item 24, a user can easily recognize information on a sample.

### (Item 25)

In the management system according to any one of Items 20 to 24, the information of the sample includes a location where the sample was collected, and
the display terminal device maps and displays the information of the sample on a map based on acquired map information and the location where the sample was collected. According to the management system of Item 25, a user can recognize on a map a location where a target sample was acquired.

### (Item 26)

An information providing system according to one aspect is an information providing system that provides the information of the sample using the management system according to any one of Items 20 to 25,
wherein the management system:
receives a request concerning provision of the information of the sample from a user;
provides the requested information of the sample to the user; and
receives a consideration for providing the information of the sample from the user. According to the information providing system of Item 26, a user can acquire information of a sample possessed by the management system in exchange for a consideration.

### (Item 27)

In the information providing system according to Item 26, the management system further:
receives the sample and the information of the sample from a collector; and
sends a consideration for the sample and the information of the sample to the collector.

According to the information providing system of Item 27, a sample collector can obtain a consideration in exchange for a sample and information of the sample.

### (Item 28)

In the information providing system according to Item 26 or Item 27, the management system further:
receives a request concerning provision of the sample from the user;
provides the requested sample to the user; and
receives a consideration for providing the sample from the user. The information providing system according to Item 28 can obtain a consideration in exchange for sending a sample to a user. Further, a user of the information providing system can acquire a sample by paying a consideration.

### (Item 29)

In the information providing system according to any one of Items 26 to 28, the management system further:
receives a request concerning provision of an analysis result of the sample from the user;
provides the requested analysis result of the sample to the user; and
receives a consideration for providing the analysis result of the sample from the user. The information providing system according to Item 29 can obtain a consideration in exchange for sending an analysis result of a sample to a user. Further, a user of the information providing system can acquire an analysis result of a sample by paying a consideration.

### (Item 30)

An information providing system according to one aspect is an information providing system that provides the information of the sample using the management system according to Item 23, wherein the management system:
receives a request concerning provision of an analysis result of the sample from a user;
generates the requested analysis result of the sample by the analysis device;
provides the requested analysis result to the user; and
receives a consideration for providing the requested analysis result of the sample from the user. The information providing system according to Item 30 analyzes a sample in response to a request from a user and sends an acquired analysis result to the user. Therefore, even when an analysis result of a sample is not registered in a server, a user can acquire an analysis result of the sample.

### (Item 31)

A method for recovering a nucleic acid according to one aspect is a method for recovering a nucleic acid present in a liquid sample, comprising: a preparation step of preparing the liquid sample; a suspension step of adding a capture agent together with a cationic compound to the prepared liquid sample to generate a suspension; and a recovery step of recovering the capture agent contained in the suspension, wherein the capture agent includes a glass fiber or silica, and the cationic compound has a secondary amino group or a quaternary amino group. According to the method for recovering a nucleic acid of Item 31, a method for recovering a nucleic acid with an improved yield can be provided.

### (Item 32)

A kit for recovering a nucleic acid according to one aspect comprises:
a sampling bag having an opening;
a filter unit engaging with the opening and having a filter; and
a storage bag capable of accommodating the filter unit. According to the recovery kit of Item 32, a burden on a user required to recover nucleic acids contained in a liquid sample can be reduced.

### (Item 33)

The recovery kit according to Item 16 may further comprise the sampling bag, the filter unit, and the storage bag according to Item 32. According to the recovery kit of Item 33, articles necessary to carry out the method for recovering a nucleic acid are provided.

### (Item 34)

The recovery kit according to Item 32 or Item 33 may further comprise a seal for attaching an identifier indicating identification information to the storage bag. According to the recovery kit of Item 34, recovered nucleic acids can be identified based on identification information.

### (Item 35)

The recovery kit according to any one of Items 32 to 34 may further comprise a discharge unit having an accommodating portion capable of accommodating the filter unit and configured to be connectable to the opening. According to the recovery kit of Item 35, a solution in the sampling bag 3 is discharged via the discharge unit.

### (Item 36)

In the recovery kit according to Item 35, the discharge unit may include a connection port connectable to a suction filter flask. According to the recovery kit of Item 36, since a solution in the sampling bag 3 is discharged by being sucked, a time required for recovering nucleic acids can be shortened.

Although embodiments and examples of the present invention have been described above, it is originally intended to combine the configurations of the respective embodiments and respective examples described above as appropriate.

The embodiments and examples disclosed this time should be considered illustrative in all respects and not restrictive. The scope of the present invention is shown not by the embodiments and examples described above but by the scope of claims, and it is intended that all modifications within the meaning and scope equivalent to the scope of claims are included.

### [Explanation of Reference Numerals]

1, 5, 6: Container, 2, 7: Filter unit, 3: Sampling bag, 4: Cap, 8: Discharge unit, 11: Composition, 21, 72: Filter, 22: Identifier, 23, 71: Housing, 23A: Opening, 23B: Opening, 24: Seal, 31: Main body portion, 32: Opening portion, 33: Cap, 42: Storage bag, 100, 101: Nucleic acid recovery kit, 200: Terminal device, 300: Server, 301: Processor, 302: Memory, 303: Interface, 400: Storage unit, 410: Storage, 420: Terminal device, 500: Analysis device, 600: Display terminal device, 700: Display screen, 1000: Management system, 2000: Providing system.

## Claims

1. A method for recovering a nucleic acid present in an environmental liquid sample, comprising:
a preparation step of preparing the environmental liquid sample;
a suspension step of adding a capture agent together with a cationic compound to the prepared environmental liquid sample to generate a suspension; and
a recovery step of recovering the capture agent contained in the suspension,
wherein the capture agent includes a glass fiber or a silica carrier, and
the cationic compound has a secondary amino group or a quaternary amino group.

2. The method for recovering a nucleic acid according to claim 1, wherein the capture agent includes a powdery glass fiber.

3. The method for recovering a nucleic acid according to claim 2, wherein an arithmetic average fiber length of the powdery glass fiber is 20 µm or more and 500 µm or less.

4. The method for recovering a nucleic acid according to claim 1 or claim 2, wherein a material of the glass fiber includes borosilicate glass.

5. The method for recovering a nucleic acid according to claim 1 or claim 2, wherein the cationic compound further has a straight-chain alkyl group having 10 to 18 carbon atoms.

6. The method for recovering a nucleic acid according to claim 1 or claim 2, wherein the cationic compound includes a compound having a quaternary amino group and having a straight-chain alkyl group having 10 or more carbon atoms, or a compound having a secondary amino group and having a bactericidal activity.

7. The method for recovering a nucleic acid according to claim 1 or claim 2, wherein the cationic compound includes at least one selected from the group consisting of benzalkonium chloride, chlorhexidine gluconate, alkyldiaminoethylglycine hydrochloride, polyhexamethylene biguanide, N-methyldodecylamine, domiphen bromide, hexadecyltrimethylammonium bromide, 1-decyl-3-methylimidazolium chloride, 1,3-didecyl-2-methylimidazolium, tetradecyltrimethylammonium, cetylpyridinium, dodecyltrimethylammonium, trimethyloctadecylammonium, benzyldimethyldodecylammonium, 1-dodecylpyridinium chloride, benzyldimethylhexadecylammonium chloride, cetylpyridinium bromide, and didecyldimethylammonium.

8. The method for recovering a nucleic acid according to claim 7, wherein the cationic compound includes benzalkonium chloride, and
a concentration of the benzalkonium chloride is 0.0005 w/v% or more and 0.05 w/v% or less based on the suspension.

9. The method for recovering a nucleic acid according to claim 1 or claim 2, wherein a mass ratio of the capture agent to the cationic compound in the suspension is 0.25 or more and 250 or less.

10. The method for recovering a nucleic acid according to claim 1 or claim 2, wherein, in the suspension step, the cationic compound is added in order to promote binding between the capture agent and the nucleic acid.

11. The method for recovering a nucleic acid according to claim 1 or claim 2, wherein the method further comprises adding a flocculant in the suspension step, and
the flocculant includes at least one selected from the group consisting of polyaluminum chloride, polysilica iron compounds, and polymer flocculants.

12. The method for recovering a nucleic acid according to claim 11, wherein the method further comprises adding triiron tetraoxide in the suspension step.

13. The method for recovering a nucleic acid according to claim 1 or claim 2, wherein the environmental liquid sample includes at least one selected from the group consisting of river water, seawater, lake water, and sewage.

14. The method for recovering a nucleic acid according to claim 1 or claim 2, wherein, in the recovery step, the capture agent is recovered by filtering the suspension.

15. A kit for recovering a nucleic acid comprising a composition used for recovering a nucleic acid contained in a liquid sample,
wherein the composition comprises:
a capture agent including a glass fiber or a silica carrier; and
a cationic compound having a secondary amino group or a quaternary amino group.

16. A kit for recovering a nucleic acid comprising:
a sampling bag having an opening;
a filter unit engaging with the opening and having a filter; and
a cap configured to form a cavity with the filter by engaging with the filter unit.

17. The kit for recovering a nucleic acid according to claim 15, further comprising the sampling bag, the filter unit, and the cap according to claim 16.

18. The kit for recovering a nucleic acid according to claim 16 or claim 17, wherein an identifier indicating identification information is attached to the filter unit.

19. A method for recovering a nucleic acid present in a liquid sample, comprising:
a preparation step of preparing the liquid sample;
a suspension step of adding a capture agent together with a cationic compound to the prepared liquid sample to generate a suspension; and
a recovery step of recovering the capture agent contained in the suspension,
wherein the capture agent includes a glass fiber or silica, and
the cationic compound has a secondary amino group or a quaternary amino group.
